(19) Europäisches Patentamt
European Patent Office
Office européen des brevets

(11) **EP 3 913 357 A1**

(12) **EUROPEAN PATENT APPLICATION**

(43) Date of publication:
**24.11.2021 Bulletin 2021/47**

(51) Int Cl.:
**_G01N 21/64_** _(2006.01)_

(21) Application number: **20176067.5**

(22) Date of filing: **22.05.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **Erasmus University Medical Center Rotterdam**
**3015 GE Rotterdam (NL)**
• **Laboratory for functional and metabolic imaging Swiss Federal Institute of Technology (EPFL)**
**1015 Lausanne (CH)**

(72) Inventors:
• **Croizat, Gauthier**
**69300 Caluire (FR)**
• **Mik, Egbert G.**
**3015 GD, Rotterdam (NL)**
• **Wagnières, Georges**
**1095 Lutry (CH)**
• **Gerelli, Emmanuel**
**74150 Vaulx (FR)**

(74) Representative: **Moré, Solveig Helga et al**
**Kroher Strobel**
**Rechts- und Patentanwälte PartmbB**
**Bavariaring 20**
**80336 München (DE)**

(54) **A METHOD AND DEVICE FOR OPTICAL QUANTIFICATION OF OXYGEN PARTIAL PRESSURE IN BIOLOGICAL TISSUES**

(57) The disclosure relates to methods and devices for monitoring the concentration of a substance, preferably oxygen, in a cell or tissue, e.g., in cells of the human skin. In particular, it provides a method for determining the concentration of a quencher, such as oxygen and/or the concentration of a probe, e.g., a heme precursor such as protoporphyrin IX (PpIX), wherein the probe is capable of exhibiting luminescence (delayed fluorescence (DF) or phosphorescence) and or transient triplet absorption, preferably, deDF, in a living cell. The method comprises steps of exciting the probe, measuring the lifetime of the luminescence exhibited by said probe, wherein, in the presence of the quencher, the lifetime is shortened as compared to the lifetime in the absence of the quencher, and correlating said lifetime with said concentration. The disclosed method leads to more precise results than conventional methods, because of adaptations based on the understanding of the influence of the concentration of the probe and its excitation fluence rate (intensity) on the analysis. For example, the simultaneous time-resolved detection of the probe excimer and monomer DF allows estimation of the probe concentration and compensation of the probe self-quenching effect in the quencher concentration calculation, increasing the measurement precision. Taking into account second order triplet interactions also permits the interpretation of non-exponential decays and further improvement of the quencher and probe concentration estimation. Disclosed methods rely, e.g., on measurement at different emission wavelengths and application of an adaptive Stern-Volmer relationship, the decay central fitting method and/or a mixed orders approach. Said methods can be applied, e.g., for bedside monitoring of patients. Also disclosed is the use of the PpIX precursor 5-aminolevulinic acid (5-ALA), or derivatives thereof, in this method, and a device suitable therefor.

Fig. 7

**Description**

The invention relates to methods and devices for monitoring the concentration of a substance, preferably, oxygen, in a cell or tissue, e.g., in cells of the human skin. In particular, it provides a method for determining the concentration of a quencher, such as oxygen and/or the concentration of probe, e.g., a heme precursor such as protoporphyrin IX (PpIX), wherein the probe is capable of exhibiting luminescence (delayed fluorescence or phosphorescence) and or transient triplet absorption, preferably, delayed fluorescence in a living cell. The method comprises steps of exciting the probe, measuring the lifetime of the luminescence, e.g., delayed fluorescence, exhibited by said probe, wherein, in the presence of the quencher, the lifetime of an excited triplet state is shortened as compared to the lifetime of an excited triplet state in the absence of the quencher, and correlating said lifetime with said concentration. The invention optimizes the method and leads to more precise results than conventional methods, because of adaptations based on the understanding of the influence of the concentration of the probe, of its triplet state, and its excitation fluence rate (intensity) and fluence on the analysis. Taking into account second order triplet interactions also permits the interpretation of non-exponential decays and further improvement of the quencher and probe concentration estimation. Methods of the invention rely, e.g., on measurement at different emission wavelengths and application of an Adaptive Stern-Volmer relationship, the decay central fitting method and/or a mixed orders approach. Said method can be applied, e.g., for assessment or imaging of tissue oxygenation, viability and/or oxygen utilization, diagnosis of conditions, assessment of treatment effects or titration of treatments, bedside monitoring of patients, for example, for intensive care patients, patients having sepsis or patients undergoing surgery, organ transplants or a tumor therapy such as radiotherapy or photodynamic therapy. The invention also relates to the use of the PpIX precursor 5-aminolevulinic acid (5-ALA), or derivatives thereof, in this method. A device suitable for the method of the invention is also provided.

Health control, diagnosis of disease and/or monitoring of treatment of disease often involves measurement of various parameters. One parameter is the concentration of a certain substance, such as oxygen, within at least part of an organism. Local tissue oxygenation is an important parameter in the diagnosis and treatment of a wide range of diseases. Measurements of the amount of oxygen present in a specific part of a subject are for instance carried out during peri-operative monitoring in the operating room and intensive care and for diagnosis of a wide range of clinical disorders in which tissue oxygenation is central to the development and cure of disease. Examples include diagnosis of cardiovascular disease, monitoring healing of decubitus and diabetic wounds, monitoring hyperbaric correction of radiation wounds and assessment of success of bypass surgery. Monitoring of tissue oxygen pressure (pO$_2$) during critical illness is considered a major need in the adequate treatment of intensive care patients (Siegemund et al., 1999). Assessment of tumor oxygenation is also of interest, as oxygen is an important determinant for success of radiotherapy and photodynamic therapy. Hence, the concentration of oxygen in a tumor is preferably assessed to determine whether radiotherapy, or photodynamic therapy, is recommended and/or to adapt the radiation or light dose. For example, if the concentration of oxygen in parts of the tumor is low (e.g., the partial oxygen pressure being below 10 mm Hg as demonstrated by Epel el al., 2019, Oxygen-Guided Radiation Therapy. Int J Radiat Oncol Biol Phys. 103(4):977-984), an enhanced local radiation can be used to increase treatment success.

Dioxygen is a molecule of utmost biological importance because of its role as the primary biological oxidant. Therefore, oxygen plays a key role in the oxidation/reduction reactions that are coupled to cellular respiration and energy supply. For instance approximatively 22 mol of dioxygen per day (0,254 $\mu$mol.s$^{-1}$) is consumed for a 70 kg 20-year-old male, where, at least 90% is reduced to water into the mitochondria for ATP production (Wagner et al., 2011, Free Radical Biology and Medicine 51(3):700-712). Adequate measurement of oxygen concentrations in biological samples like cells, tissues and whole organs is important to gain insight in the determinants of oxygen supply and utilisation under normal and pathological conditions. The clinical interest in methods providing information about blood-flow and oxygen delivery at the (sub-)organ level (e.g. microcirculatory) is growing. This is amongst other things because of increasing insight into the role of the microcirculation in pathogenesis, and the importance of adequate tissue perfusion as endpoint of treatment (Siegemund et al., 1999. Intens Care Med 25:1044-1060).

Various techniques have been developed for direct or indirect oxygen measurements in tissue, each having its specific advantages and disadvantages (J.M. Vanderkooi et al., 1991.Am J Physiol 260:C1131-50). Conventionally, measurements of tissue oxygenation have been made by use of oxygen electrodes and spectrophotometry of the hemoglobin or myoglobin molecule. Reflection spectrophotometry records the difference in absorption and scattering between a standard reference sample and a tissue sample. The method is based on the illumination of a tissue sample by light with a known spectral content and detection of the diffusely reflected light from the tissue at several different wavelengths. Drawbacks of these conventional techniques are that they are either mechanically disruptive (insertion of oxygen electrodes) or qualitative (spectrophotometry). These constrains have led to the development of alternative methods.

One of the most promising techniques in this respect has been the use of oxygen dependent quenching of phosphorescent dyes for measurements in the microcirculation (Vanderkooi et al., 1987. J Biol Chem 262:5476-5482; Vanderkooi et al., 1987. Science 236:568-569; Sinaasappel & Ince, 1996 J Appl Physiol:2297-2303; Sinaasappel et.

al., 1999. J Physiol 514: 245-253). The technique is based upon the principle that a molecule, most frequently metallo-porphyrins, that has been excited by light can either release this absorbed energy as light (phosphorescence) or transfer the absorbed energy to oxygen (without light emission). This results in an oxygen-dependent phosphorescence intensity and lifetime. The relationship between the lifetime and the oxygen concentration is usually given by the Stern-Volmer relationship (Vanderkooi, 1989. Biochim Biophys Acta 976:1-27). Calibration constants associated with the Stern-Volmer relationship allow oxygen concentrations to be calculated from the measured lifetimes. The measurement of lifetimes instead of intensities presents many advantages, in particular because it allows quantitative measurements without the influence of tissue optical properties.

Improved methods for determining the concentration of oxygen in living cells, tissue or organs, which, e.g., do not necessitate the injection of metallo-porphyrin, were developed by Mik et al. These methods are based on excitation of heme precursors such as Protoporphyrin IX (PpIX). PpIX is a natural aromatic molecule comprising four pyrrole rings produced inside the mitochondria as an intermediate in the biosynthesis of heme. PpIX is naturally present in mitochondria inside cells at relatively low concentrations. However, for measuring $pO_2$ in cells or tissues, it is advantageous to increase the endogenous synthesis of PpIX to raise its concentration to levels facilitating the analysis by measurement of delayed fluorescence lifetime of PpIX. PpIX is generated in tissue in increased amounts after administration of a precursor, e.g., 5-aminolevulinic acid or derivatives thereof, e.g., with a transdermal patch for skin applications, or systemically (e.g. with a solution administered orally, or intravenously) for various solid tumours.

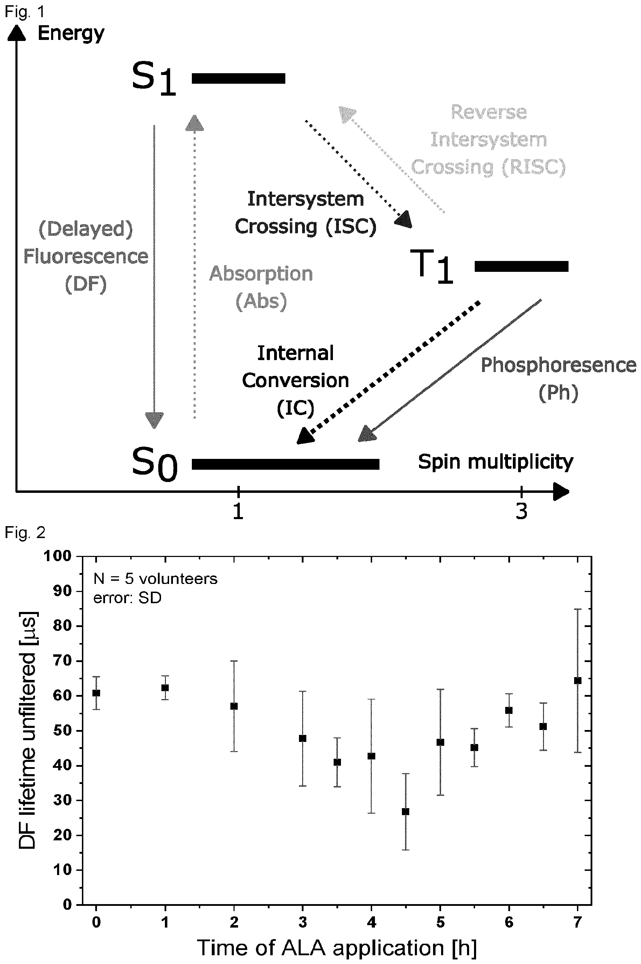

PpIX presents a strong spin-orbit coupling between the first excited singlet $S_1$ and triplet $T_1$ states (Balzani et al., 2014, Photochemistry and Photophysics. Concepts, Research, Applications. Angew. Chemie Int. Ed. 53:8817-8817, Section 3.6), and, thus a strong delayed fluorescence. Delayed fluorescence is a radiative molecular desexcitation which has the same spectrum as prompt fluorescence, but radiates at much longer time scales. It is the result of four consecutive steps, illustrated by a simplified Jablonski diagram, as shown e.g., in Fig. 1. First, a molecule in the ground state So undergoes excitation to the first excited singlet state $S_1$ through the absorption of a photon. From this state, the molecule can either desexcite to So, a transition called fluorescence if radiative, and internal conversion (IC) if not. Or it can undergo a spin-forbidden transition to the first excited triplet state $T_1$, called Intersystem Crossing (ISC). Once in the triplet state, the molecule has various pathways of desexcitation. It can go back to $S_0$: if radiative, this transition is called phosphoresence (Ph); if not, Internal Conversion again. Apart from the desexcitation to $S_0$, the molecule can also go back to $S_1$. with the help of external energy, it can experience Reverse Intersystem crossing (RISC). The subsequent radiative desexcitation to $S_0$ is called delayed fluorescence (DF). Delayed fluorescence has the same spectrum as fluorescence (because it corresponds to a desexcitation between S1 and $S_0$), but a much longer lifetime, comparable to phosphorescence, typically in the us to ms range. For example, the lifetime of DF of intracellular PpIX (in the absence of oxygen) may be up to 800 $\mu$s. Upon absorption or Intersystem Crossing, some molecules may reach higher order singlet or triplet states ($S_2$, $T_2$). These states rapidly desexcite to the first energy levels, and they are normally not considered.

According to the recited state of the art, the DF intensity ($I_{DF}$) following excitation of PpIX molecules over time ($t$) follows:

$$I_{DF}(t) = I_0 e^{-t/\tau} \qquad (1)$$

With $I_0$ the initial DF intensity, $\tau$ the first excited triplet state ($T_1$) lifetime, or, equivalently, the DF lifetime (since the fluorescence lifetime is much shorter than the $T_1$ lifetime). While in the $T_1$ state, PpIX interacts with surrounding oxygen molecules. Ground-state triplet oxygen $^3O_2$ efficiently captures the energy of $T_1$, reaching the excited and reactive singlet oxygen state $^1O_2$. This reaction, called quenching by oxygen, can be written as

$$T_1 + {}^3O_2 \xrightarrow{k_q^{O_2}} T_1 + {}^1O_2$$

and reduces the triplet lifetime. $k_q^{O_2}$, the reaction rate constant of the quenching reaction, is called the quenching constant. This quenching reaction can be used to calculate the $pO_2$ out of the DF lifetime, according to the Stern-Volmer relationship (Lakowicz, 2006, The principles of fluorescence spectroscopy Springer, p. 954):

$$pO_2(t) = \frac{1}{k_q^{O_2}} \left( \frac{1}{\tau} - \frac{1}{\tau_0} \right) \qquad\qquad (2)$$

The calculation of the partial pressure of oxygen, $pO_2$ also requires the knowledge of $\tau_0$, the $T_1$ lifetime in absence of oxygen.

As explained above, phosphorescence also is a triplet-state-based luminescence. Accordingly, instead of DF lifetime, phosphorescence lifetime can be measured, and $pO_2$ calculated on this basis. However, PpIX phosphorescence is more difficult to detect in cells in tissue. Formula (1) can be used in an analogous way.

A further option is the detection of transient triplet-state absorption (also called triplet-triplet absorption). In this case, it is not emission of light that is monitored, rather the time-resolved light absorption of excited triplet states, measured at the same time scale as DF or phosphorescence. Similarly, equation 2 can be used to calculate the $pO_2$, $\tau$ being the first excited triplet state $T_1$ lifetime in that case.

Thus, the lifetime of the luminescence and/or transient absorption exhibited by said PpIX, in particular, delayed fluorescence and/or triplet-triplet absorption, can be measured. In the presence of oxygen, the lifetime of an excited triplet state is shortened as compared to the lifetime of an excited triplet state in the absence of oxygen. Accordingly, said lifetime is correlated with said concentration, and consequently, the concentration of oxygen can be determined (EP1742038 and WO 2007/00487, Mik et al., Nature Methods 3: 939-945, 2006; Mik et al. Biophys. J. 95(8):3977-90, 2008 (incorporated herein by reference).

While, in organic solutions, second order mechanisms have been found to play a role in delayed fluorescence lifetime of PpIX (Vinklarek et al., 2017. Photochem. Photobiol. 16:507-518), it is believed in the art that the advantage of lifetime measurements in cells is the independence from the concentration of the chromophore, making quantitative measurements possible *in vivo,* where the precise concentration of said chromophore cannot be predicted (WO 2007/004873, Vinklarek et al., 2017. Photochem. Photobiol. 16:507-518).

Based on this, methods for assessment of mitochondrial function in a sample including a tissue or organ were developed, wherein the measurement of the mitochondrial oxygen consumption rate (OCR or $VO_2$), alternatively expressed as oxygen disappearance rate (ODR), in particular upon restricting or ceasing the supply of oxygen to the tissue by temporary occlusion of microvessels therein, can be used as an indicator of mitochondrial dysfunction, i.e., for assessment of health or disease (WO 2010/019041; Harms et al., 2013. Mitochondrion 13:507-514).

A device designated COMET (Cellular Oxygen METabolism) suitable for assessment of the partial pressure of oxygen in skin, e.g., for clinical measurements in patients, is commercially available from Photonics Healthcare (Utrecht, NL), and employs methods developed by Mik et al..

Thus, the correlation between the delayed fluorescence, phosphorescence or transient triplet-state absorption of probes and suitable quenchers, e.g., between the optical properties of heme precursors such as PpIX and oxygen acting as a quencher thereof, is well known.

However, the inventors noted there is a relative imprecision of measurements using the methods developed by Mik et al. under certain conditions, in particular following long ALA application, high PpIX excitation intensities or a slight change in the measurement spot. For example, the inventors observed that there were high variations with regard to the value of the DF lifetime of intracellular PpIX in absence of oxygen $\tau_0$ observed in different studies (see eq. (2)). From study to study, values from 200 to 800 μs have been reported. When measuring the DF lifetime on a volunteer at different times during the day, the DF lifetime obtained was not constant, and showed a systematic, relatively reproducible behaviour: starting at a high value after 3h of ALA application, it decreased down to a minimum around 4 to 5 h, before increasing again. A typical curve is presented on Fig. 2.

In light of these imprecisions, the inventors addressed the problem of providing still more precise methods for determining the concentration of oxygen in living cells, tissue or organs, e.g., of human patients.

This problem is solved by the present invention, e.g., by the claimed subject matter.

The invention provides a method for determining the concentration of a quencher (preferably oxygen) and/or the concentration of a probe capable of exhibiting a triplet-state based luminescence or transient triplet absorption (preferably a heme precursor such as PpIX) in a cell, wherein the cell optionally is in a tissue or organ, comprising steps of

a) exciting the probe by irradiation with light having a wavelength and a fluence rate suitable for excitation of the probe, preferably, optimized for the probe,

b) measuring the temporal evolution of the first excited triplet state of the probe, wherein, in the presence of the quencher, the triplet-state decays more quickly compared to the decay in the absence of the quencher, preferably, measuring the triplet-state based luminescence or transient triplet absorption of the probe at at least one emission or absorption wavelength, wherein, in the presence of the quencher, the triplet-state based luminescence or transient triplet absorption decays more quickly compared to the decay in the absence of the quencher, and

c) correlating said temporal evolution with said concentration(s),

wherein second order reactions involving two excited triplet states of the probe are considered.

In the context of the present invention, different pairs of probes and quenchers may be used. After excitation, probes are capable of exhibiting a triplet-state ($T_1$) based luminescence (i.e., delayed fluorescence DF or phosphorescence (PH) or transient triplet absorption (TTA). A quencher is capable of capturing the energy of $T_1$, which reduces the triplet lifetime of the probe. Thus, in the presence of a suitable quencher for the probe, the triplet-state based luminescence (e.g., delayed fluorescence or phosphorescence) or transient triplet absorption decays more quickly compared to the decay in the absence of the quencher.

For example, when the quencher is oxygen, the probe preferably is a heme precursor such as protoporphyrin IX (PpIX). Other heme precursors that are alternative to derivatives of 5-aminolevuinic acid that are precursors of PpIX, are molecules such as Proto-porphyrinogen IX. The probe can also be a mixture of heme precursors such as Proto-porphyrinogen IX and PpIX. As known in the art and described herein, oxygen also influences the luminescence emitted by the tetra-pyrols heme precursor as well as the triplet-triplet-state absorption thereof. Preferably, the temporal evolution of the delayed fluorescence is determined.

When the quencher is oxygen, alternative probes may be phosphorescent Pt(II)-and Pd(II)-porphyrins (Papkovsky DB et al., J. of Fluo, 15(4):569-584, 2005), phosphorescent Ru (II) complexes (Geddes CD, et al., 2005. Reviews in fluorescence, vol 2. Ed. Geddes C and Lakowicz JR (New York: Springer):125-151) or cyclometallated complexes of Ir(III) al (Zitova A, et al., 2010. Analytical Biochemistry, 397(2):144-151). The characteristics of exemplary probes which may be used in the present invention are given in Table 1 of Dmitriev R, Papkovsky D 2012. Cell. Mol. Life Sci., 69:2025-2039.

Other probes for measuring parameters like temperature, calcium or pH, based on spectral variations such as absorption or emission peak shifts and/or fluorescence / phosphorescence lifetime variations are known in the art (and commercially available, e.g., https://www.thermofisher.com/de/de/home/brands/molecular-probes.html). Probes and quenchers are also disclosed, e.g., by Sarder et al. (2015. Bioconjug Chem. 26(6):963-974).

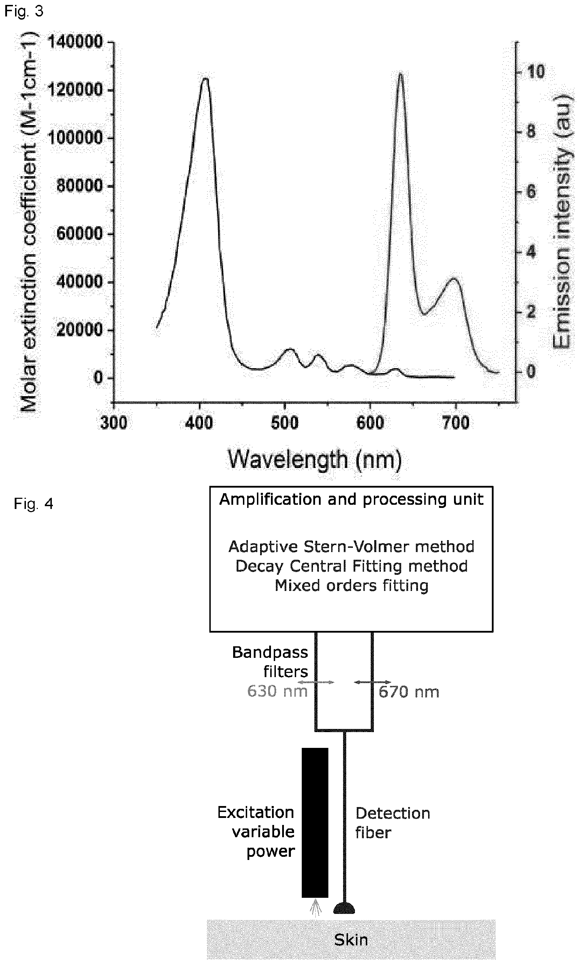

The method of the invention comprises a) exciting the probe by irradiation with light having an excitation wavelength and a fluence rate. These are chosen to correspond to the spectral properties of the probe. Excitation of the probe leads to formation of the excited triplet state of at least a fraction of the probe molecules in the cell that allows for detection of the triplet-state luminescence or transient triplet absorption. Suitable excitation wavelengths and fluence rates for the probes are known in the art. For example, the absorption spectrum of PpIX is provided in Fig. 3. PpIX can be excited at about 405nm (Soret band), about 506nm, about 515nm or about 630nm (Q bands). A wavelength means a spectral domain having a width up to several tens of nm. Excitation can be with light having a wavelength range, i.e., it is not important that the probe is excited at a single wavelength, but it can also be excited with a range of wavelengths, in other words, a spectral domain.

Typically, heme precursors such as Protoporphyrin IX are excited at a wavelength ranging between 390 and 430 nm, and/or in the range of 490-550nm, e.g., 510-530nm or about 515 nm. In the context of wavelengths, "about" means +/- 10nm. To assess the temporal evolution of the detected signals, the excitation light can be pulsed. The pulses are typically spaced with a timing that allows for full desexcitation of the system, e.g., by about 1 ms or more. However, other modes to assess the temporal evolution of the signals are possible, including high-repetition pulsing and frequency domain approaches.

The excitation fluence rate, i.e. the power per surface unit for excitation is also suitable for formation of the excited triplet state of at least a fraction of the probe molecules in the cell that allows for detection of the triplet-state based luminescence or transient triplet absorption. Typical excitation fluence, which is the time integral of the fluence rate, are well below 1 J/cm$^2$ at 405 nm, which is equivalent to approximately 10 J/cm$^2$ at 515 nm, to avoid forming PpIX photoproducts (V. Huntosova et al., 2016. Journal of Photochemistry and Photobiology B: Biology 164:49-56.).

For example, a probe in skin cells may be excited by a series of 3 to 10 27 ns pulses at 515 nm. The fluence is about 300 $\mu$J/cm$^2$, and the energy per pulse about 15 $\mu$J. In that case, the fluence rate is typically in the order of 10 KW/cm$^2$. For example, a Laser Innolas Mod3 Yb-Yag (passive cooling, frequency doubling, passive Q-switch, fiber coupling (SMA)) may be used. The excitation light may be transmitted via a light diffuser to illuminate the skin, such as a frontal diffuser producing a circular spot (e.g., with an illuminated surface of about 5 mm$^2$), with 1 ms separating each pulse. About generally means +/- one order of magnitude.

The method further comprises b) measuring the temporal evolution of the first excited triplet state of the probe, wherein, in the presence of the quencher, the triplet-state decays more quickly compared to the decay in the absence of the quencher. Thus, the triplet state lifetime is measured. The triplet state lifetime is typically assessed by measuring the triplet-state based luminescence or transient triplet absorption of the probe at at least one emission or absorption wavelength. In the presence of the quencher, the triplet-state based luminescence or transient triplet absorption decays more quickly compared to the decay in the absence of the quencher.

To assess the temporal evolution, the luminescence intensity is measured over time, i.e., at a plurality of timepoints, typically every 20-50 ns, in the relevant time range. For example, for a full resolution of faster decays, notably, during the first part of the decay at a high $pO_2$ level, every 1 - 10 ns. Repetitive measurements of such pluralities of timepoints, e.g., every ms for DF measurements, may be performed. Alternatively, a "continuous" measurement can be carried out with a detector presenting a response that is modulated at the same frequency (homodyne detection) as the excitation intensity modulation, or at a slightly different (heterodyne detection) frequency, while exciting the probe with light that is modulated in intensity at the same frequency, which usually corresponds to about the inverse of the luminescence lifetimes to measure (frequency domain-based approach). The time-domain based approach is preferred. Either way, the temporal evolution of the luminescence intensity can be assessed.

In the method of the invention, transient triplet-state absorption, or, preferably, the temporal evolution of triplet-state based luminescence selected from the group consisting of delayed fluorescence or phosphorescence may be measured. Preferably, the temporal evolution of delayed fluorescence (DF) is measured if the probe belongs to the family of PpIX. DF has the advantage of being easily detectable in comparison to phosphorescence for this family of molecules, and is easier to implement than TTA, the latter requiring for example an additional light source.

If TTA is measured, two light sources are used. The pump beam excites the probe to the $T_1$ state by emitting light at one of its absorption peaks detailed above. The probe beam, usually white light, is used to measure the evolution of the concentration of $T_1$. Its intensity is continuously measured at one of the excited triplet states $T_1$ molecules absorption peaks. These wavelengths depend on the probe. For example, for PpIX, $T_1$ absorption wavelength may be at 450 nm (Chantrell et al., 1976. Journal of Luminescence 12-13:887-891).

If triplet-state based luminescence is measured, the emission of said luminescence, e.g., delayed fluorescence in the case of the PpIX family, is measured. The emission is measured at emission wavelengths dependent on the probe. If the probe is such that, in certain condition of excitation fluence rate, probe and quencher concentration, it forms dimers out of two excited triplet states $T_1$, so-called excimers, then both the luminescence of the probe monomer and excimer may be measured. For example, for delayed fluorescence of PpIX, emission may be measured at emission wavelengths provided in Fig. 3, e.g., at about 630 nm for the monomer or about 670 nm for the excimer. Emission may be measured at a single wavelength. Reference to an emission wavelength can include reference to an emission wavelength range, in other words, a spectral domain. Preferably, emission of delayed fluorescence or phosphorescence of PpIX is detected at a wavelength in the range of 610-650 nm, e.g., 620-640 nm or about 630 nm, or in the range of 650-730 nm, e.g., 660-710 nm or about 670 nm.

In step c) of the method of the invention, said temporal evolution is correlated with said concentration of the probe and/or the quencher. The correlation is typically based on the Stern-Volmer relationship known in the art, however, in contrast to prior art methods, second order reactions (or processes) involving two excited triplet states of the probe are considered.

Several processes on which the lifetime of the excited triplet states, and consequently, e.g., delayed fluorescence, depends generally have been described in literature (but not yet in cells in tissues or organs) (Scholz and Dedic, 2016. Singlet-Oxygen-sensitized Delayed Fluorescence, 63-81; Parker and Hatchard, 1963, Transactions of the Faraday Society 59, 284; Parker, 1968. Photoluminescence of Solutions: With Applications to Photochemistry and Analytical Chemistry, Elsevier Publishing Group University of California, 2nd edn, p. 544). The necessary energy to perform RISC can come from different origins:

* The simplest DF process is called E-type DF (Eosin-type DF, also designated by Thermally Activated DF: TADF), and is activated by temperature: thermic energy provokes the RISC. The corresponding reaction is: $T_1 \xrightarrow{k_B T} S_1$ followed by $S_1 \xrightarrow{h\nu} S_0$. It is a first order reaction, which dominates at low chromophore concentration or low exciting light intensity.

* At higher concentration or higher excitation intensity, the concentration of excited triplet states $T_1$ increases: two triplet states can collide, and give rise to one excited $S_1$ molecule, and one relaxed to the ground state, following the reaction: $2T_1 \rightarrow S_1 + S_0$, followed by DF: $S_1 \xrightarrow{h\nu} S_0$. This type of DF is called P-type DF (pyrene-type DF, also called Triplet-triplet annihilation). It is a second order reaction, as two $T_1$ molecules are involved.

* Another type of DF, Singlet Oxygen Feedback DF or SOFDF, has been reported (Scholz and Dedic, 2016. Singlet-Oxygen-sensitized Delayed Fluorescence, 63-81; Vinklarek et al., 2017. Photochem. Photobiol. 16:507-518). In the experimental conditions considered, the inventors could show that SOFDF was not observed in human skin, as described in the experimental section below.

Surprisingly, in contrast to the belief held in the state of the art (e.g., EP1742038 and WO 2007/00487, Mik et al., Nature Methods 3: 939-945, 2006; Mik et al. Biophys. J. 95(8):3977-90, 2008; Mik et al., 2013. Anesthesia and Analegesia 117: 834-846; Vinklarek et al., 2017. Photochem. Photobiol. 16:507-518 with an explicit discussion of the situation in cells), the inventors could show that in cells, in particular, in skin, the temporal evolution of the triplet state, measurable e.g., by delayed fluorescence, involves a significant part of second order reactions.

In human skin, the assumption in the state of the art was that the main Reverse InterSystem Crossing (RISC) mechanism is E-type, thermal DF. According to this model, and using the notations introduced in Fig. 1 and equation 2, the DF intensity (understood as a power, an energy per unit time) due to E-type DF is: (3)

$$I_{DF}^{E}(t) = I_0 e^{-\left(k_E + k_{IC} + k_q^{O_2} pO_2\right)t} = I_0 e^{-t/\tau} \tag{3}$$

Thus, the DF lifetime is $\tau = \dfrac{1}{k_E + k_{IC} + k_q^{O_2} pO_2}$ and the DF lifetime in absence of oxygen $\tau_0 = \dfrac{1}{k_E + k_{IC}}$

Note that, for PpIX, the contribution of phosphorescence ($k_{Ph}$) is negligible at body or room temperature. PpIX phosphorescence can be measured in non-physiological environments, according to Gouterman et al., 1974. Journal of Molecular Spectroscopy 53(1):88-100.

Consequently, the unexpected results mentioned above and described in more detail in the examples cannot be explained by the state of the art model of PpIX DF:

- The DF lifetime in absence of oxygen $\tau_0 = \dfrac{1}{k_E + k_{IC}}$ was held to be the function of two reaction rates of PpIX excited states and should not vary too significantly in different environments: 200 to 800 $\mu s$ seems too big a spread.

- On a healthy volunteer, time average of the intracellular $pO2$ is expected to not be varying too significantly through long time measurement. Consequently, the time average of the DF lifetime $\tau = \dfrac{1}{k_E + k_{IC} + k_q^{O_2} pO_2}$ should also be close to constant.

Even if noisy, it should not present a strong systematic variation, as seen in Fig. 2.

In light of these surprising observations, and in light of advances in the understanding of PpIX DF *in vitro,* the inventors provide a solution to these incoherencies by taking second order reactions involving two excited triplet states of the probe into account, in particular, taking second order desexcitation into account. As shown below, the inventors surprisingly found that formation of excimers and/or triplet-triplet annihilation (e.g., P-type DF) are second order processes that, if taken into account, render the method for determining the concentration of a probe and/or a quencher in a cell more precise.

The inventors have described new biological, chemical and physical principles of the intracellular $pO_2$ measurement method based on the delayed fluorescence of Protoporphyrin IX, further developing the Triplet State Lifetime Technique (TSLT).

In particular, the discovery of the existence of PpIX excimers *in vivo,* in particular in cells in tissues or organs such as human skin, changes the understanding of DF dynamics. Excimers lead to a detectable DF signal at 670nm. Furthermore, the inventors derived a new model of triplet population evolution and associated DF signals, when second-order process play a significant role. The framework introduced, namely the concepts of depopulation equation, and measurement / depopulation pathways, predicted non-monoexponential and non-multiexponential decays. These predictions were confirmed *in vivo.*

Based on these new elements, the inventors proposed several methods to improve the precision and reliability of $pO_2$ measurements: In different aspects, new fitting procedures are provided, either in the time range considered (Decay Central Fitting method), or in the function fitted (Mixed-orders decay fitting), or in the parameters used (Adaptive Stern-Volmer relationship). The method based on the adaptive Stern-Volmer relationship is based on the specific detection of the DF signal at 630 and 670 nm. These methods are further explained below.

While the inventors' experimental observations rely on delayed fluorescence measurements, the same principles apply for methods of the invention based on phosphorescence and transient triplet absorption. Indeed, the improved $pO_2$ measurement methods are ultimately based on the excited triplet state concentration evolution analysis: any experimental method able to access it is fit for this invention.

In a preferred embodiment, the invention provides a method for determining the concentration of oxygen and/or the concentration of a heme precursor, such as PpIX, in a cell, wherein the cell optionally is in a tissue or organ, comprising steps of

a) exciting the heme precursor by irradiation with light having a wavelength and a fluence rate suitable for excitation of the probe, preferably optimized for the probe (e.g., as described above for PpIX),

b) measuring the temporal evolution of the triplet-state based luminescence or transient triplet absorption of the heme precursor at at least one emission or absorption wavelength (e.g., as described above for PpIX), wherein, in the presence of oxygen, the triplet-state based luminescence or transient triplet absorption decays more quickly compared to the decay in the absence of oxygen, and

c) correlating said temporal evolution with said concentration,

wherein second order reactions involving two excited triplet states of the heme precursor are considered.

In most embodiments of the invention, preferable, the concentration of the quencher is determined, in particular, the concentration of oxygen, in particular, if the status of a tissue, an organ or a patient is to be assessed.

However, in case photodynamic treatment, e.g., of cancer cells is desired, there also is a high interest in also determining the concentration of heme precursors such as PpIX in cells, so in that case, the method is preferably carried out to determine both the concentration of the probe, in particular, of heme precursors such as PpIX, and of the quencher, in particular oxygen. The method allows the user to determine whether the probe and quencher concentrations are in the correct range to perform photodynamic therapy. Additionally, it allows to choose the optimal excitation intensity given the probe and quencher concentration to reach the best photodynamic effect, for example the optimal level of singlet oxygen creation. If the PpIX concentration is high, a low light dose should be used. If the PpIX concentration is low, a high light does should be used. The administration of probe precursor, for example ALA, or derivatives thereof, in the case of PpIX, can also be adjusted depending on the probe concentration measured. Appropriate average PpIX concentration for photodynamic therapy ranges from 10 uM to 5 mM.

There are also forms of porphyria which result in a pathologic accumulation of PpIX in many organs, including the skin. In these cases, e.g., for diagnostic applications, the method of the invention may also be used to determine the concentration of heme precursors such as PpIX in cells.

The invention provides a method for determining the concentration of oxygen in a cell, wherein the cell optionally is in a tissue or organ, comprising steps of

a) exciting a heme precursor such as PpIX by irradiation with light having a wavelength (e.g., as defined above for PpIX) and a fluence rate suitable for excitation of the probe,

b) measuring the temporal evolution of the triplet-state based luminescence or transient triplet absorption of the heme precursor at at least one emission or absorption wavelength, wherein, in the presence of oxygen, the triplet-state based luminescence or transient triplet absorption decays more quickly compared to the decay in the absence of oxygen, and

c) correlating said temporal evolution with said concentration,

wherein second order reactions involving two excited triplet states of the heme precursor are considered. Preferably, the temporal evolution of delayed fluorescence is determined.

Alternatively, the invention provides a method for determining the concentration of a heme precursor, such as those involved in the heme biosynthetic pathway leading to the production of PpIX, in a cell, wherein the cell optionally is in a tissue or organ, comprising steps of

a) exciting the heme precursor by irradiation with light having a wavelength (e.g., as defined above for PpIX) and a fluence rate suitable for excitation of the probe,

b) measuring the temporal evolution of the triplet-state based luminescence or transient triplet absorption of the heme precursor at at least one emission or absorption wavelength, wherein, in the presence of oxygen, the triplet-state based luminescence or transient triplet absorption decays more quickly compared to the decay in the absence of oxygen, and

c) correlating said temporal evolution with said concentration,

wherein second order reactions involving two excited triplet states of the heme precursor are considered. Preferably, the temporal evolution of delayed fluorescence is determined.

In preferred embodiments of the invention, the concentration of the probe, e.g., a heme precursor such as PpIX is determined, and, optionally, then, the concentration of the quencher, e.g., oxygen, is determined. Thus, the method can serve to determine both the concentration of the probe and the quencher in the cell. In the context of the invention, concentration can also be measured as partial pressure.

As described herein, inventors have surprisingly found that second order reactions of the probe have a significant influence on the decay of the excited triplet state of the probe after excitation. Second order reactions are influenced by the concentration of excited triplet states of the probe. The concentration of excited triplet states of the probe also depends on the excitation intensity and the concentration of the probe.

Thus, in one aspect, the concentration of excited triplet states of the probe is considered for determining the concentration of the probe or the concentration of the quencher.

In a preferred method of the invention, the emission of delayed fluorescence is measured at at least two wavelengths (including two wavelength ranges, as described above), wherein these two wavelengths are suitable for distinguishing delayed fluorescence of probe monomers and of probe excimers. In a preferred embodiment, the probe is a heme precursor such as PpIX, and the first wavelength is in the range of 615-645 nm, optionally, about 630 nm and the second wavelength is in the range of 646 nm-700 nm, optionally, about 670 nm. The emission at the two wavelengths is preferably measured essentially simultaneously, i.e., without any undue delay, and preferably in the same sample volume. Preferably, it is thus measured by two detectors, however, one detector can also be used if the control unit is configured to measure at both wavelengths in close sequence.

Based on this, the adaptive Stern-Volmer relationship may be used for determining the concentration of the quencher. Keeping in mind the central results discovered by the inventors: first, that PpIX lifetime, particularly in absence of oxygen, strongly depends on the local PpIX concentration, in a Stern-Volmer like quenching relationship, and second, that the ratio of DF energy at 670 and 630 nm is linearly linked to the local PpIX concentration.

The adaptive Stern-Volmer relationship

i) calculates the concentration of the probe based on the ratio of the probe excimer DF energy to the probe monomer DF energy

ii) calculates the delayed fluorescence lifetime in absence of quencher at the probe concentration calculated in step i), and

iii) calculates the quencher concentration by feeding the DF lifetime in absence of oxygen calculated at step ii) into the Stern-Volmer relationship, thus adapting the Stern-Volmer relationship to the probe concentration,

wherein, preferably, the probe is a heme precursor such as PpIX and the ratio $E_{670}/E_{630}$ is used in step i).

In more details the method steps comprise:

- calculating the DF energy at the monomer and excimer wavelenghts: $E_{630} = \int_0^{+\infty} I_{DF}^{630}(t)\, dt$ and

$$E_{670} = \int_0^{+\infty} I_{DF}^{670}(t)\, dt$$

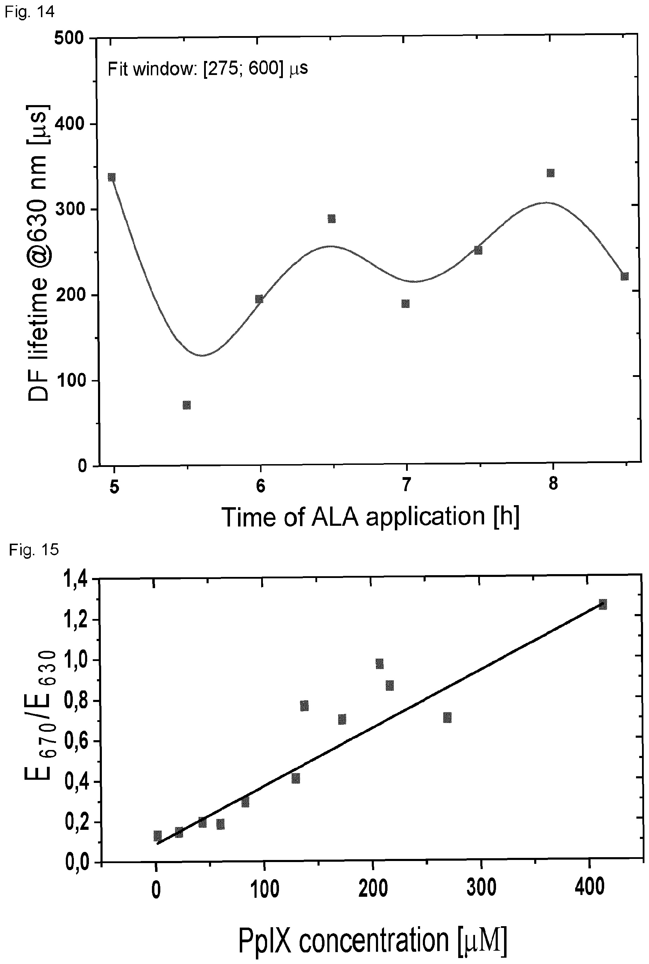

- deducing the concentration of PpIX ([PpIX]) in the cells, wherein the ratio $E_{670}/E_{630}$ is a linear function of the PpIX concentration: $E_{670}/E_{630} = a + b[PpIX]$ with a and b known parameters (see Fig. 15),

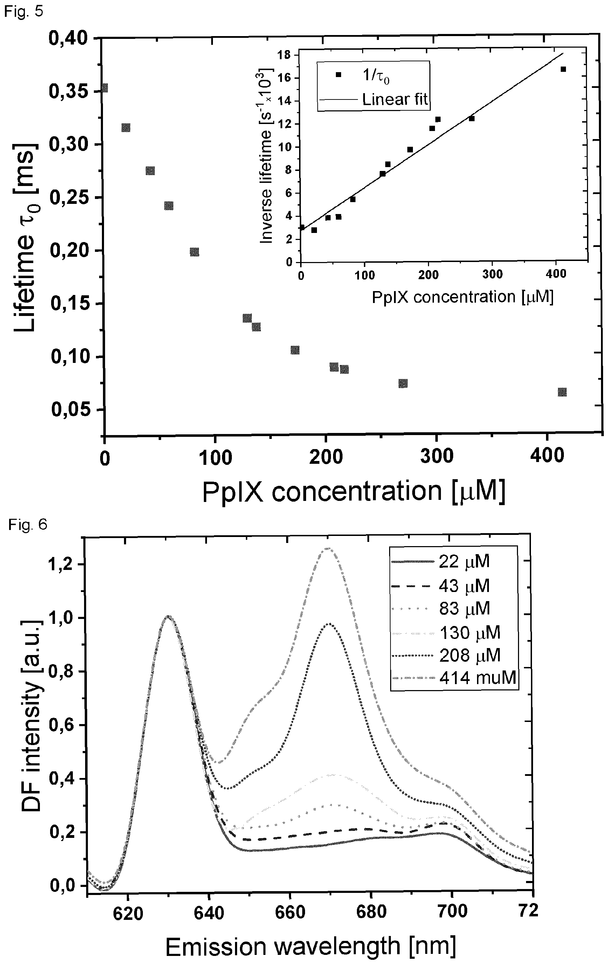

- determining the DF lifetime at 630 nm in absence of oxygen $\tau_0^{630}$ from the concentration of PpIX, according to Fig. 5,

$$I_{DF}^{630} = I_0 e^{-\frac{t}{\tau_{630}}} + S_0$$

- fitting the best mono-exponential function to the decay at 630 nm: by determining the most adapted values for $I_0$, $\tau_{630}$ and $S_0$

- deducing the $pO_2$ using the adaptive Stern-Volmer relationship ( $k_q^{O_2}$ the quenching constant by oxygen, is known):

$$pO_2 = \frac{1}{k_q^{O_2}}(\frac{1}{\tau_{630}} - \frac{1}{\tau_0^{630}})$$

Note: the "adaptive" nature of this relationship lies in the fact that it does not use a single value for $\tau_0^{630}$, but it is adapted depending on the local concentration of PpIX.

In one aspect of the invention, which can be combined with other approaches, e.g., with the approach underlying the adaptive Stern-Volmer relationship, the decay central fitting method or the mixed orders decay as explained in more detail below, the fluence rate (intensity) of the light for excitation is considered for determining the concentration of the quencher. If a low fluence rate is used, the excited triplet state is reached for a low fraction of the probe molecules. If a high fluence rate is used, the excited triplet state is reached for a high fraction of the probe molecules. The fluence rate thus directly affects the concentration of excited triplet state in the cells.

Thus, in one embodiment of the method of the invention, the probe is excited by irradiation with light having at least two different fluence rates (intensities) or light doses (if the pulse durations are shorter than the deexcitation times). Variable excitation allows for more precise determination of the contribution of first vs second-order measurement pathways e.g., as explained in detail in the examples below. Indeed, for a given excitation light dose, first order processes are proportional to the exciting fluence rate (intensities), while second-order processes are proportional to the square of the fluence rate (intensities). For instance, the relative contribution of E-type and P-type DF can be determined with this method.

Alternatively or additionally, the probe can also be excited by irradiation with light having at least two excitation wavelengths. Like the excitation fluence rate and dose, the wavelength also directly influences the fraction of probe molecules in an excited triplet state, and, thus, the concentration of the excited triplet state. For example, excitation at a wavelength corresponding to a maximum of the probe absorption peaks leads to more energy being transferred to the probe.

However, the excitation wavelength also has effects on the location of the volume of cells for which the determination is carried out, because different wavelengths have different penetration depths. Thus, the excitation wavelength can also be varied in order to analyse the concentration of the probe and/or the concentration of quencher at different penetration depths. The penetration depths of light of different wavelengths are known in the art (S. Jacques, "Optical Properties of Biological Tissues: A Review", Physics in Medicine and Biology 58(11):R37-R61, 2013.). If this is of interest, the intensity of excitation can be adapted to make up for lower excitation.

In another aspect, which is optionally combined with excitation at variable excitation intensities or doses, the decay central fitting method (DCF) is used for correlating said temporal evolution with said concentration of quencher. In particular, the decay central fitting method rejects the initial non-exponential part of the decay. Preferably, the method further rejects the tail of the decay dominated by noise, thus selecting the central part of the decay of delayed fluorescence intensity over time. The central part of the decay of delayed fluorescence intensity over time comprises the time after $t_1$, when the monoexponentiality indicator first gets below a threshold T, and, optionally, before $t_2$, when the signal to noise ratio gets below a threshold $T$.

In details, the algorithm determines threshold values and time boundaries as follows: Assuming that the DF intensity $I(t)$ can be written as the sum of noise $\Delta I(t)$, centered around 0, and an average intensity $\bar{I}(t)$, which is typically the function obtained after fitting of the experimental signal, such that $I(t) = \bar{I}(t) + \Delta I(t)$.

Defining the monoexponentiality indicator as

$$M(t) = (1 - \frac{\bar{I}''\bar{I}}{(\bar{I}')^2})^2$$

With ' denoting the derivation with respect to time. The thresholds are values such that: $M(t_1) = 0.7 = T$.

Introducing the signal to noise ration SNR(t), the noise dominated section starts, for example, at the time value

$$SNR(t_2) = \frac{\bar{I}(t_2) + \Delta I(t_2)}{\bar{I}(t_2)} = 2 = T'$$

such that                                                     In detail, the algorithm comprises:

- determining $t_1$ and $t_2$ as detailed herein

- between $t_1$ and $t_2$, fitting a mono-exponential to $I(t) = I_0 e^{-\frac{t}{\tau_{630}}} + y_0$ by determining the most adapted values for $I_0, \tau_{630}$ and $y_0$,

- deducing $pO_2$ using the conventional Stern-Volmer relationship ( $k_q^{O_2}$, the quenching constant by oxygen, and $\tau_0^{630}$, the DF lifetime for $pO_2$=0 and $[PpIX]{\approx}0$, are known):

$$pO_2 = \frac{1}{k_q^{O_2}} \left( \frac{1}{\tau_{630}} - \frac{1}{\tau_{630}^0} \right)$$

For example, under the conditions used in the COMET setup, the mono-exponential linear section comprises the time between 275 and 600 $\mu$s.

The DCF method may be used in combination with a variation of excitation intensities to quantify linear vs. quadratic behaviour, in particular, the contribution of P-type DF and excimer DF.

In another aspect of the invention, called "mixed-orders fitting", the concentration of the quencher is determined on the basis of the assumption that the excited triplet state of the probe deactivates through a mix of first and second order reactions. The deactivation of the excited triplet state of the probe can be determined by measurements of delayed fluorescence, phosphorescence or TTA.

Preferably, the concentration of the quencher is determined on the basis of the assumption that the delayed fluorescence is caused by a mix of first and second order reactions.

Thus, the concentration of the quencher may be determined based on the formula:

$$I_{630}(t) = \theta_{S_1}\left( k_E [T_1]_{mixed} + k_P [T_1]_{mixed}^{\,2} \right) + y_0$$

With

$$[T_1]_{mixed}(t) = \frac{k_E + k_{IC} + k_q^{O_2} pO_2}{\left( \dfrac{k_E + k_{IC} + k_q^{O_2} pO_2}{[T_1]_0} + 2(k_P + k^d) \right) e^{\left( k_E + k_{IC} + k_q^{O_2} pO_2 \right)t} - 2(k_P + k^d)}$$

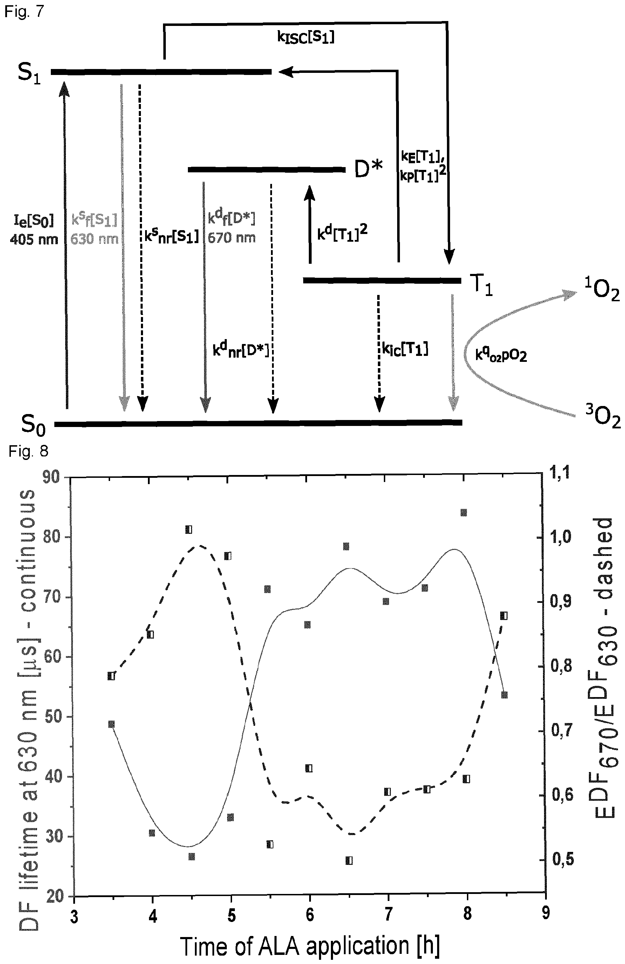

wherein $k_E, k_{IC}$, $k_q^{O_2}$ and $k^d$ are known parameters (see, e.g., Fig. 7), $\theta_{S_1}$ is the fluorescence quantum yield of the probe $S_1$ state, and $[T_1]_0$, $pO_2$, and $y_0$ shall be fitted.

Throughout the invention, if the probe is a heme precursor such as PpIX and the quencher is oxygen, before step a) of the method of the invention, a precursor of PpIX, preferably, 5-aminolevulinic acid (ALA), is administered to the cell(s) or has been administered to the cell(s). Instead of ALA, derivatives, in particular, prodrugs thereof may also be used, e.g., esters such as 5-ALA-OMe or 5-ALA-OHex. ALA prodrugs and derivatives are known in the art (e.g., Tewari et al., 2018. Photochemical & Photobiological Sciences 17:1553-1572; Wu et al., 2017. ACS Appl Mater Interfaces 9(17):14596-14605, Li et al., 2019. European Jounral of Medicinal Chemistry 181:111582). This increases the level of PpIX in the cells, even though PpIX is also naturally present at low concentrations, as heme precursor. PpIX is typically produced in the mitochondria of cells. Alternatively, the level of PpIX can be increased by administering PpIX. Different methods for administering PpIX, or preferably, ALA and its derivatives are known in the art. The selection depends on the cells of interest, in particular, on the tissue or organ. For example, ALA can be administered to skin in a transdermal patch (e.g., available from Photonamics, Wedel, DE).

The invention thus also provides a precursor of protoporphyrin IX, preferably, 5-aminolevulinic acid or, alternatively, prodrugs thereof, for use of the invention in an *in vivo* method, wherein the probe is a heme precursor, such as PpIX, and the quencher is oxygen and the cell is in a human or animal subject, preferably, a human subject.

It is clinically very useful that the method of the invention can be used for assessment of mitochondrial function in cells. In particular, the invention provides a method for assessment of mitochondrial function in a sample comprising cells in a tissue or organ, comprising restricting or ceasing the supply of oxygen to said sample and carrying out the method of the invention with said sample, wherein the probe is PpIX and the quencher is oxygen. The method can be carried out as disclosed in WO 2010/01041 with the adaptations described herein, e.g., measuring at two emission wavelengths and using the adaptive Stern-Volmer relationship.

Throughout the invention, cell encompasses at least one cell. Typically, the concentration of probe and/or quencher is determined in a plurality of cells. The cells of interest are determined by the sample for which the emission of luminescence and/or TTA is assessed.

The cell typically is a living cell. It may also be a dying cell.

Preferably, the cell is part of a tissue or organ. The tissue may be part of an organ, or it may be an isolated tissue. Due to the easy accessibility of skin, the cell preferably is part of skin. However, e.g., under perioperative conditions, other tissues or organs are also accessible, e.g., a tumor. The organ may for example also be liver, kidney, brain, bladder or heart. The tissue may be tumor tissue.

Preferably, the cell is *in situ* in a subject, preferably, a patient, e.g., a human subject or patient. The cell may also be in a tissue or organ intended for transplantation, wherein preferably, the method provides information that allows for assessment of the viability of the tissue or organ. For example, a tissue or organ derived from a subject, e.g., a human subject may be transported, and before transplantation to the recipient, the status, e.g., the concentration or partial pressure of oxygen in the tissue or organ may be assessed. This allows for conclusions on the viability, and thus, depending on the assessment, the tissue or organ is transplanted or rejected.

The invention also provides a method for assessment of the status of a patient, e.g., a sepsis patient, a critically ill patient, a patient undergoing a tumor treatment (e.g., phototherapy or photodynamic therapy), a patient undergoing surgery, a patient suffering from a neurodegenerative condition, such as Alzheimer, Parkinson or Huntington diseases, or a decubitus patient, comprising carrying out the method of the invention, wherein the cell is a cell of said patient. Preferably, the patient is human.

The invention also provides a method of treating a patient, optionally, a human patient, e.g., a sepsis patient, a critically ill patient, a patient undergoing a tumor treatment (e.g., phototherapy or photodynamic therapy), a patient undergoing surgery, a patient suffering from a neurodegenerative condition, such as Alzheimer, Parkinson or Huntington diseases, or a decubitus patient, or for selecting an organ suitable for transplantation to a patient, comprising carrying out the method of any of the invention, wherein the cell is a cell of said patient (or, for selecting an organ, a cell of said organ).

For example, in the context of photodynamic therapy, ALA is applied, and converted to PpIX in a tumor. This can be advantageously combined with the method of the invention, e.g., to find out if a certain kind of treatment, e.g., irradiation or photodynamic treatment is suitable for treatment of the tumor. For instance, based on the oxygen concentration determined using the invention, the light dose to perform photodynamic therapy can be adapted to reach maximal efficiency. For instance if little oxygen is present in (parts of) the tumor, additional local irradiation is likely to improve the treatment effect. Oxygen concentration in a tumor and/or around a tumor site can also be monitored with a method of the invention, to monitor the progress of disease and/or therapy. The treatment can then be adapted to the progress.

In a further aspect, the invention provides a method for determining the temperature at a location of PpIX in a cell, preferably, in the mitochondria of a cell, comprising steps of

a) minimizing the concentration of a quencher, preferably, by restricting or ceasing the supply of oxygen to said cell,

b) exciting a probe capable of exhibiting delayed fluorescence by irradiation with light having a wavelength and a fluence rate suitable for excitation of the probe, wherein the probe preferably is a heme precursor such as PpIX,

c) determining the lifetime of the monoexponential component of the decay according to a method of any of embodiments 1-36, preferably, by the decay central fitting method, or by the mixed-orders fitting method, and

d) calculating the temperature through the Arrhenius relationship.

A method of the invention for determining the temperature in the mitochondria of a cell, may comprise steps of

- reducing the concentration of triplet quencher to 0 (e.g., for $pO_2$ measurement, e.g., in human skin, press the tissue (e.g., skin) until the oxygen is removed and the DF lifetime is stable);

- determining the sample DF at 630 nm;

- by using the decay central fitting method (DCF), estimating the lifetime of the monoexponential component of the decay $\tau$, in seconds,
wherein $\tau$ is linked to the sample temperature T through the Arrhenius relationship:

$$T = \frac{E_a}{R} \ln(A\tau)$$

wherein:

$E_a$ is the energy gap between the first excited singlet and triplet states of PpIX ($S_1$ and $T_1$ respectively, typically $E_a = 35 \, kJ.mol^{-1}$; Suisalu, et al. Phosphorescence-detected triplet state microwave resonance of a protoporphyrin molecule in n-octane, Chem. Phys. Lett. 101 (1983) 182-186; Gouterman, et al. Porphyrin free base phosphorescence, J. Mol. Spectrosc. 53 (1974) 88-100.)

$R = 8.314 \, J. \, K^{-1}. \, mol^{-1}$ the ideal gas constant

$A$: $[s^{-1}]$ the E-type reaction rate constant pre-exponential factor: $k_E = Ae^{-\left(\frac{E_a}{RT}\right)}$

A temperature variation can be determined even more precisely through the following relationship, which does not require the knowledge of A:

$$T_2 - T_1 = \frac{E_a}{R} \ln\left(\tau_2 / \tau_1\right)$$

In one aspect, the invention provides a device suitable for carrying out the method of the invention, comprising

- an excitation light source arranged to illuminate a sample volume, optionally with a variable excitation power, and
- a light detector arranged for the time-resolved detection of luminescence, such as fluorescence or phosphorescence, or transient triplet absorption by the sample volume, optionally from both the monomer and the excimer form of the probe simultaneously, and
- a control unit configured to obtain measurements of luminescence, such as fluorescence or phosphorescence, or transient triplet absorption by the sample volume.

The excitation light is provided by an excitation light source, which can be any light source capable of providing light of a, or several, suitable wavelength(s) in continuous, modulated or pulsed fashion, wherein, continuous light is combined with an accessory presenting variable transmission. Examples include a xenon light source with bandpass filter or monochromator, light emitting diodes (LEDs) and several types of laser systems (e.g., diode lasers or other solid state or gas lasers, tunable or not). Typically, the light source is a flash lamp, a LED or pulsed laser system.

The light detector may be any photodetector, such as for instance, a photodiode, avalanche photodiode, photomultiplier tube, charge coupled device (e.g., CCD camera). The light detector may comprise an image intensifier or may not comprise an image intensifier. Detection systems may comprise phase-locked detection techniques in order to improve the signal-to-noise ratio. The light detector may be arranged to detect light emission, e.g., luminescence, as it is functionally linked to an optical fiber capturing said light emission, e.g., luminescence. A system of lenses, filters and/or mirrors may also be used to capture the light, e.g., luminescence from the cell. If an optical fiber is used, it is preferably positioned with one end facing the measurement sample comprising the cell or cells of interest, in contact or not, and the other end towards the detector.

For detection of delayed fluorescence, the photodetector is typically gated with a tunable delay to exclude the strong prompt fluorescence and only capture the delayed fluorescence.

The control unit may be configured to obtain repetitive or continuous measurements of the luminescence or triplet triplet absorption. As stated above, the detected parameter preferably is delayed fluorescence, so the device is preferably suitable for measuring delayed fluorescence. Devices comprising these features are known in the state of the art, e.g, the COMET device (Photonics Healthcare).

Based on the modifications to prior art methods for determining more precise concentrations of probe and/or quencher, the device also needs to be modified by at least one feature. A device of the invention thus further comprises

i) means for determining the delayed fluorescence lifetimes and intensities of a probe simultaneously at two emission wavelengths suitable for distinguishing delayed fluorescence or phosphorescence (preferably, delayed fluorescence) of probe monomers and/or of probe excimers; and/or

ii) means for exciting the probe with one or several different wavelengths, fluence and/or fluence rates.

For example, bandpass filters suitable for selecting delayed fluorescence at a wavelength (or wavelength range) suitable for distinguishing the delayed fluorescence of probe monomers and excimers, e.g., at about 630 nm and at about 670 nm for PpIX as the probe, may be employed. Alternatively, or in addition, the excitation light source, e.g., the excitation laser may have a variable excitation power and/or pulse duration. The $pO_2$ calculation algorithm may also be changing to a calculation algorithm of the invention. A device with preferred features is presented in Fig. 4.

A preferred device of the invention comprises means for determining the delayed fluorescence lifetimes and/or intensities of a probe simultaneously at two emission wavelengths suitable for distinguishing delayed fluorescence of probe monomers and of probe excimers, wherein the same sample is analysed. If the probe is a heme precursor such as PpIX, and delayed fluorescence is determined, the first detected wavelength is in the range of 615-645 nm, optionally, about 630 nm, and the second wavelength is in the range of 646 nm-700 nm, optionally, about 670 nm. For example, the means for determining the delayed fluorescence of PpIX at two emission wavelengths may be spectral filters, grating or spectrometers, preferably, spectral filters such as band-pass filters.

The device of the invention may comprise means for sequential or simultaneous light detection at at least two different wavelengths by a light detector, e.g., a photomultiplier, and a photomultiplier array. The device may, e.g., comprise means for simultaneous light detection.

The device of the invention may comprise a gated photomultiplier that is configured to reject the initial prompt fluorescence, and then opens to collect the delayed fluorescence, or two such gated photomultipliers.

Alternatively, or additionally, the device of the invention may comprise means for exciting the probe with at least two, preferably, five different excitation fluence rates and/or fluence, optionally, at least 10. The means for exciting PpIX with at least two, preferably, five different excitation fluence rates and/or fluence may be, e.g. filters suitable for placement in the excitation beam, a laser with variable power and energy per pulse, and/or a plurality of lasers with different excitation fluence rates, preferably, a laser with variable excitation power.

The device of the invention is useful for carrying out the method of the invention, e.g., it is suitable for assessing the concentration of a probe and/or quencher in a cell, typically in a tissue or organ, preferably, *in situ* in a human patient.

Preferably, the device of the invention further comprises a pressure pad for applying local pressure on tissue containing arterioles, veins and/or capillary bed that supply oxygen to the sample volume. This allows for assessment of the status of the mitochondria in said tissue or sample volume, as described herein.

The device of the invention may further comprise

iii) means for exciting the probe with at least two different excitation wavelengths. Such means may comprise spectral filters, grating, a light source, e.g., a laser, with variable excitation wavelength, or two or more lasers with different excitation wavelengths, or spectrometers, preferably, several lasers.

The device of the invention preferably further comprises a processing unit capable of processing the obtained measurements and configured to apply the adaptive Stern-Volmer relationship and/or the decay central fitting method and/or the mixed orders fitting method, preferably, at least the adaptive Stern-Volmer relationship. Alternatively, the device may comprise communication means adapted to communicate with an external processing unit capable of processing the obtained measurements and configured to apply the adaptive Stern-Volmer relationship and/or the decay central fitting method and/or the mixed orders fitting method, preferably, at least the adaptive Stern-Volmer relationship, e.g., a computer.

In one aspect, the invention provides the use of the device of the invention for determining the concentration of a quencher such as oxygen and/or of a probe such as PpIX in a cell according to the method of the invention.

Embodiments

In embodiment 1, the invention provides a method for determining the concentration of a quencher (preferably oxygen) and/or the concentration of a probe capable of exhibiting a triplet-state based luminescence or transient triplet absorption (preferably a heme precursor such as PpIX) in a cell, wherein the cell optionally is in a tissue or organ, comprising steps of

a) exciting the probe by irradiation with light having a wavelength and a fluence rate suitable for excitation of the probe,

b) measuring the temporal evolution of the triplet-state based luminescence or transient triplet absorption of the probe at at least one emission or absorption wavelength, wherein, in the presence of the quencher, the triplet-state based luminescence or transient triplet absorption decays more quickly compared to the decay in the absence of the quencher, and

c) correlating said temporal evolution with said concentration(s),

wherein second order reactions involving two excited triplet states of the probe are considered.

In embodiment 2, in the method of embodiment 1, the quencher is oxygen, and the probe is a heme precursor such as protoporphyrin IX (PpIX).

In embodiment 3, in the method of any of embodiments 1 or 2, the temporal evolution of delayed fluorescence of the probe is measured at at least one emission wavelength.

In embodiment 4, in the method of any of embodiments 1 or 2, the temporal evolution of phosphorescence of the probe is measured at at least one emission wavelength.

In embodiment 5, in the method of any of embodiments 1 or 2, the temporal evolution of transient triplet absorption of the probe is measured at at least one absorption wavelength.

In embodiment 6, in the method of any of embodiments 1-5, the concentration of the quencher is determined, preferably, the concentration of oxygen.

In embodiment 7, in the method of any of embodiments 1-6, the concentration of the probe is determined, preferably, the concentration of heme precursors such as PpIX.

In embodiment 8, in the method of any of embodiments 1-7, the concentration of the probe is determined, and, then, the concentration of the quencher is determined, preferably, the concentration of heme precursors such as PpIX and of oxygen.

In embodiment 9, in the method of any of embodiments 1-8, wherein the concentration of excited triplet states of the probe is considered for determining the concentration of the probe and/or the concentration of the quencher.

In embodiment 10, in the method of any of embodiments 1-9, the emission of the delayed fluorescence is measured at at least two emission wavelengths, wherein these two emission wavelengths are suitable for distinguishing delayed fluorescence of probe monomers and of probe excimers.

In embodiment 11, in the method of any of embodiments 1-10, the probe is a heme precursor such as PpIX and the first emission wavelength is in the range of 615-645 nm, optionally, about 630 nm and the second emission wavelength is in the range of 646 nm-700 nm, optionally, about 670 nm.

In embodiment 12, in the method of any of embodiments 1-11, the adaptive Stern-Volmer relationship is used for determining the concentration of the quencher.

In embodiment 13, in the method of any of embodiments 1-12, the method

i) calculates the concentration of the probe based on the ratio of probe eximer DF energy to the probe monomer DF energy,

ii) calculates the delayed fluorescence lifetime in absence of quencher at the concentration of the probe calculated in step i), and

iii) calculates the concentration of the quencher based on the delayed fluorescence lifetime in the absence of quencher calculated in step ii), in particular, by feeding the DF lifetime in absence of oxygen calculated at step ii) into the Stern-Volmer relationship, thus adapting the Stern-Volmer relationship to the probe concentration,

wherein, preferably, the probe is a heme precursor such as PpIX and the ratio $E_{670}/E_{630}$ is used in step i).

In embodiment 14, in the method of any of embodiments 1-13, the fluence rate (intensity) of the light for excitation is considered for determining the concentration of the quencher. In embodiment 15, in the method of embodiment 14, the probe is excited by irradiation with light having at least two different fluence rates.

In embodiment 16, in the method of any of embodiments 1-7, the decay central fitting method is used for correlating said temporal evolution with said concentration of quencher, wherein the decay central fitting method rejects the initial non-exponential part of the decay.

In embodiment 17, in the method of embodiment 16, the method further rejects the tail of the decay dominated by noise, thus selecting the central part of the decay of delayed fluorescence intensity over time.

In embodiment 18, in the method of embodiment 17, the central part of the decay of delayed fluorescence intensity over time comprises the time after $t_1$, when the mono-exponentiality indicator first gets below a threshold T, and, optionally, before $t_2$, when the signal to noise ratio gets below a threshold $T$.

In embodiment 19, the method of any of embodiments 16-18 is used in combination with a variation of excitation intensities to quantify linear vs. quadratic behaviour, in particular, the contribution of P-type DF and excimer DF.

In embodiment 20, in the method of any of embodiments 1-7, the concentration of the quencher is determined on the basis of the assumption that the excited triplet state of the probe deactivates through a mix of first and second order reactions, wherein, preferably, said deactivation is determined by delayed fluorescence or phosphorescence measurements or measurements of TTA.

In embodiment 21, in the method of any of embodiments 1-7 or 20, the concentration of the quencher is determined on the basis of the assumption that the delayed fluorescence is caused by a mix of first and second order reactions.

In embodiment 22, in the method of any of embodiments 1-7 or 20-21, the concentration of the quencher is determined based on the formula:

$$I_{630}(t) = \theta_{S_1}\left(k_E[T_1]_{mixed} + k_P[T_1]_{mixed}{}^2\right) + y_0$$

wherein

$$[T_1]_{mixed}(t) = \frac{k_E + k_{IC} + k_q^{O_2}pO_2}{\left(\frac{k_E + k_{IC} + k_q^{O_2}pO_2}{[T_1]_0} + 2(k_P + k^d)\right)e^{\left(k_E + k_{IC} + k_q^{O_2}pO_2\right)t} - 2(k_P + k^d)}$$

In embodiment 23, in the method of any of embodiments 1-22, the probe is a heme precursor such as PpIX and it is excited by irradiation with light having a wavelength of about 515 nm.

In embodiment 24, in the method of any of embodiments 1-23, the probe is a heme precursor such as PpIX and it is excited by irradiation with light having a wavelength of about 405 nm, wherein, preferably, the signal to noise ratio is improved compared to a measurement according to embodiment 23.

In embodiment 25, in the method of any of embodiments 1-24, the probe is excited by irradiation with light having at least two different excitation wavelengths.

In embodiment 26, in the method of any of embodiments 1-25, the selection of the excitation wavelength allows for selection of the penetration depth of the excitation light and, consequently, for selection of the analysed cells or tissue layers.

In embodiment 27, in the method of any of embodiments 1-26, the probe is a heme precursor such as PpIX and the quencher is oxygen, and, before step a), a precursor of PpIX, preferably, 5-aminolevulinic acid or derivatives thereof, is administered to the cell or has been administered to the cell.

In embodiment 28, the invention provides a method for assessment of mitochondrial function in a sample comprising a tissue or organ, comprising restricting or ceasing the supply of oxygen to said sample and carrying out the method of any of embodiments 1-27 with said sample, wherein the probe is PpIX and the quencher is oxygen.

In embodiment 29, in the method of any of embodiments 1-28, the cell is a living cell.

In embodiment 30, in the method of any of embodiments 1-29, the cell is part of a tissue.

In embodiment 31, in the method of any of embodiments 1-30, the cell is part of an organ.

In embodiment 32, in the method of any of embodiments 1-31, the cell is part of skin.

In embodiment 33, in the method of any of embodiments 1-32, the cell is *in situ* in a patient.

In embodiment 34, in the method of embodiment 1-33, the patient is human.

In embodiment 35, in the method of any of embodiments 1-34, the cell is in a tissue or organ intended for transplantation, wherein preferably, the method provides information that allows for assessment of the viability of the tissue or organ.

In embodiment 36, the invention provides a method for assessment of the status of a patient selected from the group comprising a sepsis patient, a critically ill patient, a patient undergoing a tumor treatment (e.g., phototherapy or photodynamic therapy), a patient undergoing surgery, a patient suffering from a neurodegenerative condition, such as Alzheimer, Parkinson or Huntington diseases, or a decubitus patient, or for assessment of the status of an organ potentially suitable for transplantation to a patient, comprising carrying out the method of any of embodiments 1-35, wherein the cell is a cell of said patient or organ.

In embodiment 37, the invention provides a method of treating a patient, optionally, a human patient, selected from the group comprising a sepsis patient, a critically ill patient, a patient undergoing a tumor treatment (e.g., phototherapy or photodynamic therapy), a patient undergoing surgery, a patient suffering from a neurodegenerative condition, such as Alzheimer, Parkinson or Huntington diseases, or a decubitus patient, or for selecting an organ suitable for transplantation to a patient, comprising carrying out the method of any of any of embodiments 1-36, wherein the cell is a cell of said patient or organ.

In embodiment 38, the patient in the method of any of embodiments 36 or 37 is a human patient.

In embodiment 39, the invention provides a method for determining the temperature at a location of PpIX in a cell, preferably, in the mitochondria in a cell, comprising steps of

a) minimizing the concentration of a quencher, preferably, by restricting or ceasing the supply of oxygen to said cell,

b) exciting a probe capable of exhibiting delayed fluorescence by irradiation with light having an excitation wavelength and a fluence rate suitable for excitation of the probe, wherein the probe preferably is a heme precursor such as PpIX,

c) determining the lifetime of the monoexponential component of the decay according to a method of any of embodiments 1-36, preferably, by the decay central fitting method, and

d) calculating the temperature through the Arrhenius relationship.

In embodiment 40, the invention provides a precursor of protoporphyrin IX, preferably, 5-aminolevulinic acid, for use in an *in vivo* method of any of embodiments 1-39, wherein the probe is a heme precursor such as PpIX and the quencher is oxygen and the cell is in a human or animal subject.

In embodiment 41, the invention provides a device suitable for carrying out the method of any of embodiments 1-39, comprising

- an excitation light source arranged to illuminate a sample volume, and
- a time-resolved light detector arranged to detect luminescence, such as fluorescence or phosphorescence, or transient triplet absorption by the sample volume, and
- a control unit configured to obtain measurements of luminescence, such as fluorescence or phosphorescence, from or transient triplet absorption by the sample volume,

wherein said device comprises

i) means for determining the delayed fluorescence of a probe at two emission wavelengths suitable for distinguishing delayed fluorescence of probe monomers and of probe excimers, optionally, simultaneously; and/or

ii) means for exciting the probe with at least two, optionally, five different excitation fluence rates.

In embodiment 42, the device of embodiment 41 comprises means for determining the delayed fluorescence of a probe at two emission wavelengths suitable for distinguishing delayed fluorescence of probe monomers and of probe excimers, wherein preferably the device is suitable for simultaneous time-resolved detection of monomer and excimer delayed fluorescence.

In embodiment 43, in the device of embodiments 42, the probe is a heme precursor such as PpIX, delayed fluorescence is determined, and the first wavelength is in the range of 615-645 nm, optionally, about 630 nm and the second wavelength is in the range of 646 nm-700 nm, optionally, about 670 nm.

In embodiment 44, in the device of any of embodiments 41-43, the means for determining the delayed fluorescence lifetimes and intensities of PpIX at two wavelengths are selected from the group comprising spectral filters, grating and spectrometers, preferably, spectral filters such as band-pass filters.

In embodiment 45, the device of any of embodiments 41-44 further comprises means for sequential or simultaneous light detection at at least two different wavelengths by a light detector, e.g., photomultipliers.

In embodiment 46, the device of embodiment 45 comprises means for simultaneous light detection.

In embodiment 47, in the device of any of embodiments 41-46 comprises a gated photomultiplier that is configured to reject the initial prompt fluorescence, and then opened to collect the delayed fluorescence.

In embodiment 48, the device of any of embodiments 41-47 comprises means for exciting the probe with at least two, preferably five different excitation fluence rates and/or fluences.

In embodiment 49, in the device of embodiment 48, the means for exciting PpIX with at least two, e.g., five different excitation fluence rates and/or fluences are selected from the group consisting of filters suitable for placement in the excitation beam, a laser with variable excitation power and energy per pulse, and a plurality of lasers with different excitation fluence rates and/or fluences.

In embodiment 50, the device of any of embodiments 41-49 further comprises a pressure pad for applying local pressure on tissue containing arterioles, veins and/or capillary bed that supply oxygen to the sample volume.

In embodiment 51, the device of any of embodiments 41-50 further comprises
iii) means for exciting the probe with at least two different excitation wavelengths.

In embodiment 52, in the device of embodiment 51, the means for exciting PpIX with at least two different excitation wavelengths comprise spectral filters, grating, several light sources, e.g., a laser, with variable excitation wavelengths, or two or more lasers with different excitation wavelengths, and spectrometers, preferably, spectral filters such as band-pass filters.

In embodiment 53, the device of any of embodiments 41-52 further comprises a processing unit capable of processing the obtained measurements and configured to apply the adaptive Stern-Volmer relationship and/or the decay central fitting method and/or the mixed orders fitting method, preferably, at least the adaptive Stern-Volmer relationship.

In embodiment 54, the invention provides the use of the device of any of embodiments 41-53 for determining the concentration of a quencher such as oxygen and/or of a probe such as PpIX in a cell according to the method of any of embodiments 1-35.

*Legends*

Fig. 1 Simplified Jablonski diagram of delayed fluorescence, with the name of the corresponding processes. Dotted line: non-radiative, continuous line: radiative. This diagram characterizes the excited states of molecules: on the y-axis, the energy of the outer orbit electrons is represented, and on the x-axis the configuration of the spin these electrons. Singlet state: antiparallel spins, spin multiplicity 1. Triplet state: parallel spins, spin multiplity 3.

Fig. 2 Evolution of the DF lifetime $\tau$ as a function of time on healthy volunteers' sternum. The DF was collected on the whole [600; 750] nm range. There is a distinct minimum around 4-5 h

Fig. 3 Absorption (black, left curve and axis) and emission (grey, right curve and axis) spectrum of PpIX. PpIX can be excited at 405 (Soret band), 506, 515 or 630nm (Q-bands) depending on the excitation penetration and excited states concentration desired. PpIX fluorescence is mainly detectable around 630nm.

Fig. 4 One option for an improved intracellular oxygen measurement device. This version could include a variable excitation laser, which would allow the quantification of first and second-order processes. The excitation diameter would be large compared to the penetration length (about 100 $\mu$M), to minimize variations in the excitation fluence rate. It would also include bandpass filters at 630 and 670 nm, to distinguish between monomer and excimer DF. Finally, it would include a new pO$_2$ calculation algorithm: preferably the adaptive Stern-Volmer method, but the Central Decay Fitting and Mixed-orders methods are also possible.

Fig. 5 Evolution of $\tau_0$ with [PpIX]. We observe a decrease, following the equation

$$\frac{1}{\tau_0} = 36.8[PpIX] + 2.74 \times 10^3,$$

$\tau_0$ in s and [PpIX] in $\mu$M. We have identified a Stern-Volmer like self-quenching of PpIX of constant $k_q^{PpIX} = 36.8\ \mu M^{-1} s^{-1}$.

Fig. 6 Evolution of the DF spectrum with PpIX concentration, in absence of oxygen. A broad, featureless, red shifted (compared to the 630 nm peak) band develops at 670nm.

Fig. 7 Updated Jablonski diagram taking excimers into account. Excimers form according to $2T_1 \to D^*$ and subsequently desexcite at 670 nm. Their lifetime is comparable to that of $S_1$, as the DF at 630 and 670 nm are detected simultaneously.

Fig. 8 Compared evolution of $\dfrac{E_{DF}^{670}}{E_{DF}^{630}}$ and $\tau_{630}$ over time, on one specific patient. The anticorrelation is clear, strongly suggesting the presence of excimers *in vivo.* This shows that the local PpIX concentration induces a strong noise-like variation of the lifetime which is not related to physiology. Averaging such graphs for different volunteers is not relevant here: as the variation of the energy ratio / $\tau_{630}$ depends on the local PpIX concentration, it strongly depends on the probe positioning, and is relatively variable between patients. The variation of $\dfrac{E_{DF}^{670}}{E_{DF}^{630}}$ reflects more local PpIX inhomogeneities than average PpIX concentration trends, contrary to figure 2 which includes the whole [600;750] nm window).

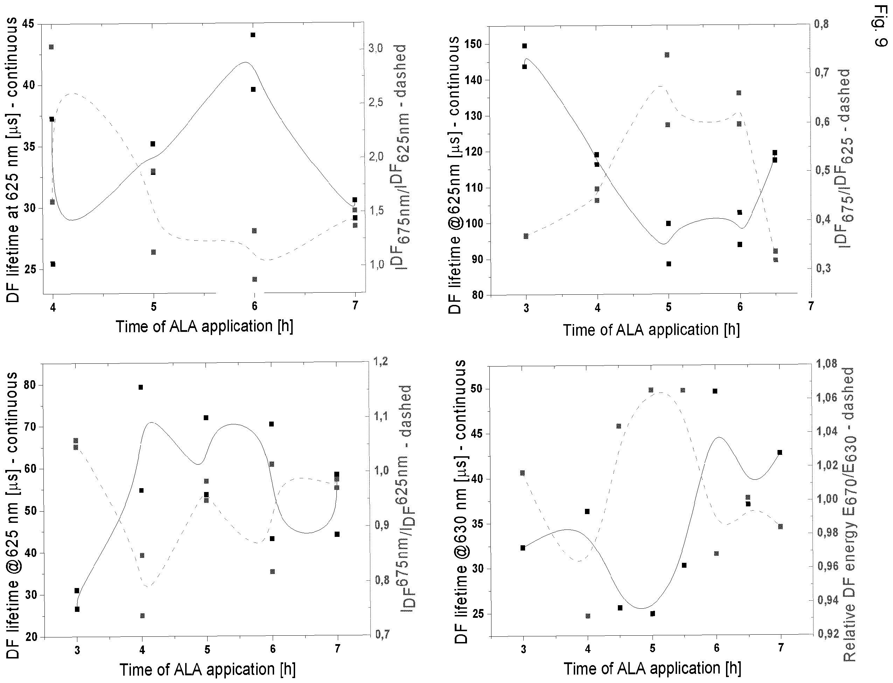

Fig. 9 Confirmation of the anticorrelation of $\tau_{630}$ with $\dfrac{E_{DF}^{670}}{E_{DF}^{630}}$ on 4 other volunteers. The anticorrelation is visible in every graph. The data presented on the bottom left hand corner was obtained while pressing the skin (pO$_2$=0).

Continuous line; DF lifetime 625 nm, Dashed line $\dfrac{E_{DF}^{670}}{E_{DF}^{630}}.$

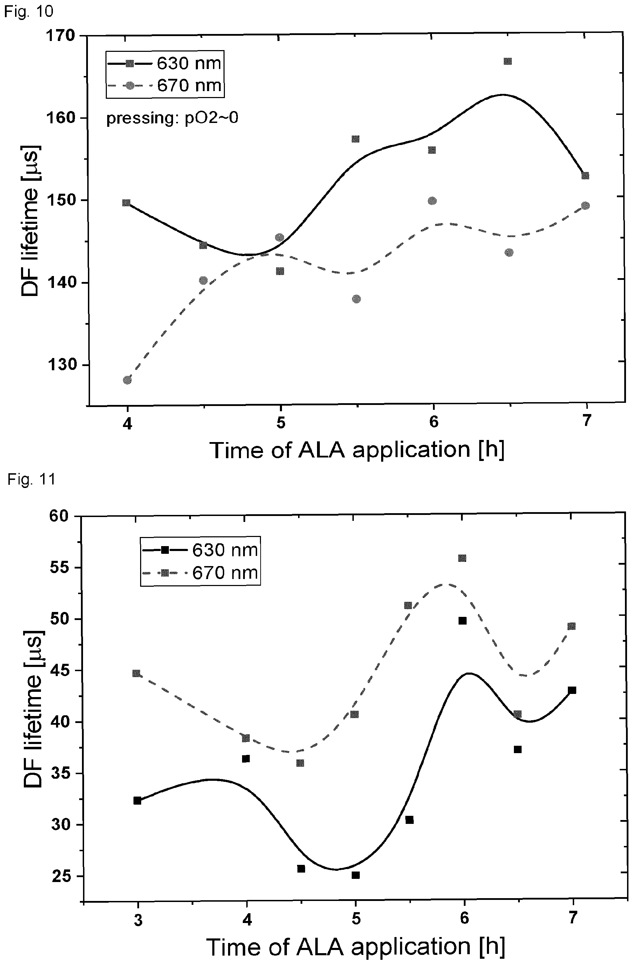

Fig. 10 Compared evolution of $\tau_{670}$ and $\tau_{630}$ over time, as the skin is **compressed** to reach pO$_2$~0. The anticorrelation is visible, confirming the existence of two competitive fluorescence process at 670 and 630 nm, thus further proving the existence of excimers. The repeatability of a lifetime measurement in our conditions is around 20%.

Fig. 11 Compared evolution of $\tau_{670}$ and $\tau_{630}$ over time in the standard **uncompressed** case. The lifetimes are clearly correlated, as they are defined by the amount of oxygen quenching. Observations are coherent with our new model.

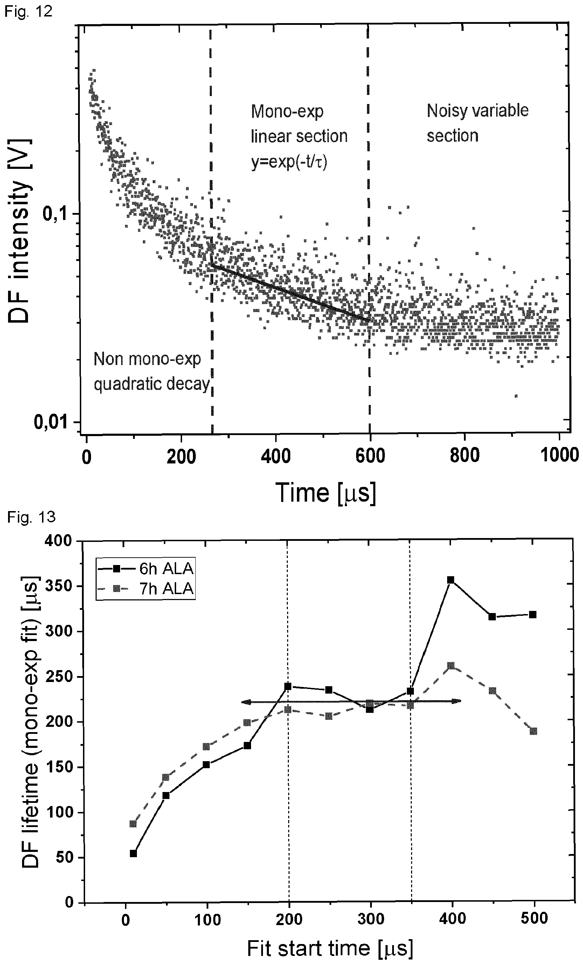

Fig. 12 Splitting of a DF signal (semilogscale) according to different kinetic behaviour: non mono-exponential initially (not linear on the graph), followed by a linear, $[T_1]_0$ independent monoexponential decay, and finally noise

Fig. 13 Evolution of the DF lifetime fitted by the Origin algorithm as a function of the fitting range lower boundary $t_1$. Clearly, the quadratic region is visible as the lifetime increases sharply before 200 $\mu$s, and differs between datatsets. Then the mono-exponential region appears with a plateau between 200 $\mu$s and 350 $\mu$s. The plateau region is not only relatively flat, but also close between datasets, which were measured at different times in the day: it is free of the influence of differences in PpIX concentration. Lastly, after 350 $\mu$s, erratic values due to noise are visible.

Fig. 14 DF lifetime at 630 nm (uncompressed measurement, 8 h ALA) fitted on the reduced interval [275; 600] $\mu$s, in accordance with the DCF method. Although the curve is less erratic than on Fig. 2, it is not as constant as expected.

Fig. 15 Relationship between the DF energy ratio at 670 and 630 nm $\dfrac{E_{DF}^{670}}{E_{DF}^{630}}$ and the PpIX concentration, as measured in vitro. The curve is linear: $\dfrac{E_{DF}^{670}}{E_{DF}^{630}} = 0.09 + 2.5 \times$ 10$^{-3}$[PpIX], [PpIX] in $\mu$M, r$^2$=0.94. This linearity is characteristic of excimer DF.

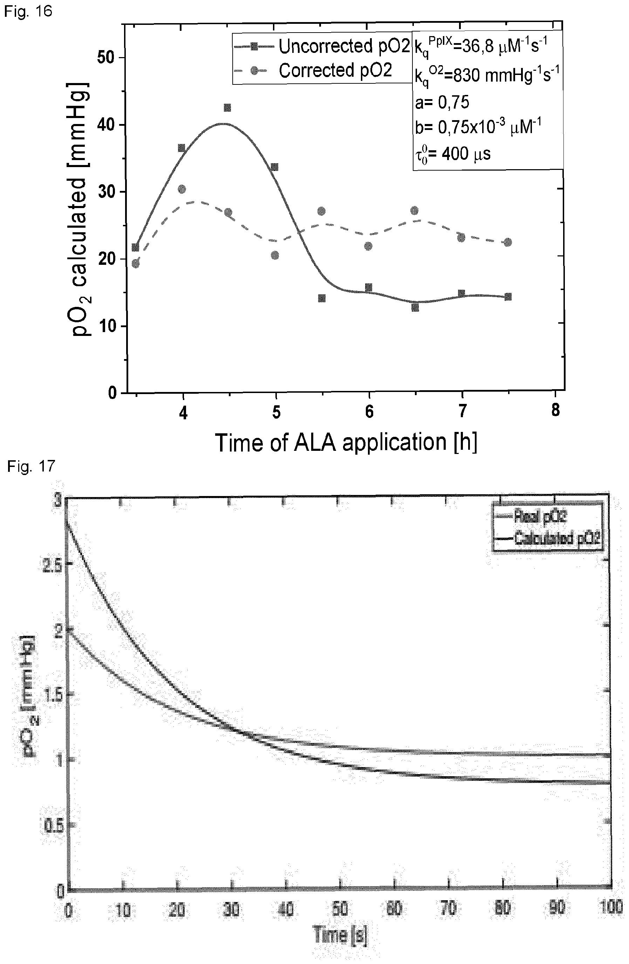

Fig. 16 Comparison between the $pO_2$ as calculated by the standard Stern-Volmer equation (uncorrected $pO_2$ and the adaptative SV equation (corrected $pO_2$), on the sternum of a volunteer. The correction reduces the $pO_2$ spread from [10;43] mmHg to [18;30] mmHg. Parameters: $k_q^{PpIX}$ and $\tau_0^0 \rightarrow$ PpIX PpIX self-quenching constant and lifetime in absence of PpIX, as defined by $\dfrac{1}{\tau_0^{630}} = \dfrac{1}{\tau_0^0} + k_q^{PpIX}[PpIX]$ (defined in Fig. 5); $a, b \rightarrow$ linear link between energy ratio and PpIX concentration: $\dfrac{E_{DF}^{670}}{E_{DF}^{630}} = a + b[PpIX]$ (defined in Fig. 15).

Fig. 17 Influence of fitting an incorrect model of PpIX DF (mono-exponential decay + Standard Stern-Volmer) on the $pO_2$ measured in an oxygen consumption experiment. The two curves are very similar in their outlook: an improper understanding of the DF of PpIX produces values that are only slightly different from reality (real $pO_2$: curve starts at *2 mmHg*).

## Examples

### *In vitro discovery of PpIX excimers*

### Measurement & Methods

The inventors worked with a solution of Protoporphyrin IX (PpIX, Fluka, > 99.5%) diluted in Dimethylformamide (DMF, Sigma Aldrich D4551, > 99%). Concentration ranged from 1 to 400 $\mu$M. The solution was placed in a 1cm wide quartz cuvette. Delayed fluorescence curves were acquired on a calibrated Horiba Fluorolog-3 spectrofluorometer, operated in front face mode. The cuvette was illuminated by a pulsed Xenon lamp (pulse duration FWHM: 1 $\mu$s, ≈ 10000 pulses per curve), filtered using a monochromator. The sample was excited at 405 nm, with a FWHM of 5 nm. The emission at different wavelengths was collected with a FWHM of 3 nm. The temperature of the setup was maintained at 32°C (or piloted when temperature was the variable) using a Pelletier Carry PCB-150 thermoregulator. The $pO_2$ was brought to zero by bubbling $N_2$ in the cuvette at a flow rate of 3L/min, using a Brick™ gas mixer. The zone of bubbling was not illuminated by the excitation beam, and the samples were constantly stirred during measurements. The excitation intensity was modified by placing neutral density filters in the excitation beam. Exponential decays were fitted using Origin V8 (OriginLab, Northampton, Massachusetts): in the expression of a mono-exponential decay

$$(t) = I_0 \exp\left(-\frac{t}{\tau}\right) + y_0 \,,$$ the initial intensity $I_0$, the DF lifetime $\tau$, and the offset $y_0$ were determined using the built-in Levenberg-Marquardt algorithm. The delayed fluorescence signals were collected and fitted from 0,13 to 11 ms.

**Results**

The first and simplest experiment was to quantify the DF lifetime in absence of oxygen as a function of the concentration of PpIX. In theory, $$\tau_0 = \frac{1}{k_E + k_{IC}}$$ should be independent of the concentration of PpIX ([PpIX]). The curve is presented on Fig. 5. Contrary to our expectations, the inventors however observed a self-quenching phenomenon: the higher the concentration of PpIX, the smaller the lifetime: as the solution gets crowded, PpIX molecules stay a shorter time in the triplet state $T_1$. This result is in complete disagreement with the established model of PpIX DF. While, so far, dependency on the concentration of PpIX in cells has not yet been observed or postulated, the inventors considered it could explain the three incoherencies they have identified earlier:

- The observed variations of $\tau_0$ in different studies could simply be that the parametrization (reducing the $pO_2$ to 0) was done at different concentrations of PpIX, thus there were different self-quenching levels. This is plausible, as the intracellular concentration of PpIX is hard to control *in vivo.*
  - The decrease of $\tau$, measured in the skin up to 4-5 h, could be due to the increasing intracellular concentration of PpIX, due to the transformation of ALA in the skin into PpIX, causing self-quenching, and thus reducing the lifetime. Later, when PpIX starts to be catabolized, its concentration decreases and $\tau$ increases again.
- Finally, on patients, when the ALA application was done the evening before surgery, [PpIX] reaches its maximum before the first measurement, and is steadily decreasing during the day: the device misinterprets the increase of $\tau$ for a decrease of $pO_2$, while in reality, it could simply be a reduction of the self-quenching effect. Combining the two quenching relationships (eq. 2 and Fig. 5), we obtain the following link between $pO_2$ and [PpIX] variations: The rate of PpIX catabolization in human cells:

$$\frac{\partial[PpIX]}{\partial t} = \frac{k_q^{O_2}}{k_q^{PpIX}} \frac{\partial pO_2}{\partial t}$$

was estimated by inventors as on average, -226 $\mu$M/h.

Conversely, by looking at the decrease of DF lifetime between 2 and 4-5 hours of PpIX application, the inventors could extract the average DF lifetime variation:

$$\frac{\partial \tau}{\partial t} \in [-10; -5] \, \mu s.h^{-1}$$

which gives a PpIX concentration rate of:

$$\frac{\partial[PpIX]}{\partial t} = -\frac{1}{k_q^{PpIX} \bar{\tau}^2} \frac{\partial \tau}{\partial t} \in [85; 170] \, \mu M.h^{-1} \qquad (4)$$

These rates are surprisingly high, suggesting that the concentration in cells could easily be above mM. Interestingly, other methods of oxygen sensing using porphyrin nanoparticles have reported unexplained quenching effects, which the inventors postulate to be based on the same mechanism (Kubat et al., 2017. ACS Applied Materials and Interfaces 9: 36229-36238).

A second question is the molecular origin of the self-quenching. By measuring the DF spectrum of different concentrations of PpIX solutions, it appeared that a peak at 670 nm was gradually developing as concentration increased, as presented on Fig. 6. The change of spectrum shows that another desexcitation process than $S_1 \rightarrow S_0$ takes place at high concentration. This added to similar observations made in the past on pyrene (Parker et al., 1968; Birks et al.,

1966. Spectrochimica Acta 22: 323-331) allowed to conclude to the existence of dimers of excited $T_1$ molecules, so called excimers.

The formation and desexcitation of excimers is presented on an updated Jablonski diagram (Fig. 7). While these *in vitro* studies do not allow for the conclusion that the same mechanism applies in cells, the inventors set out to find out if this was the case, and if it could account for the inconsistencies encountered. The question was whether excimers also form *in vivo:* the environment around PpIX molecules is so different from the homogeneous, freely diffusing *in vitro* environment, that their existence has to be proven before any conclusions can be drawn.

## *Assessement of the existence of PpIX excimers in vivo and contribution of P-type Delayed Fluorescence*

### Materials & Methods

### "Dumbo" setup

Plasters of 8mg of ALA (Alacare 8mg medicated plaster, Photonamic GmbH, Wedel) were applied on the skin of 20 healthy volunteers, in a mediothoracic position, on the sternum. The 2x2 cm$^2$ plasters contain a suspension of 5-ALA HC1 crystals suspended in a polymer matrix. The delayed fluorescence of PpIX was readily detectable from 3 to > 15 h of ALA application, with a maximum intensity around 8 h. The outer layer of dead skin cells (stratum corneum) was removed using an abrasion pad, following the standard microdermabrasion method (Mik et al., 2013). Every hour, from 3 to 8 h of ALA application, the DF in the skin was quantified using a dedicated optical setup. The skin was excited by a series of 3 to 10 27 ns pulses at 515 nm (light doses $\approx$ 300 $\mu$J/cm$^2$. Energy per pulse: $\approx$ 15 $\mu$J according to Innolas Service Test sheet Inno P1166, 18/02/2016. Laser Innolas Mod3 Yb-Yag, passive cooling, frequency doubling, passive Q-switch, fiber coupling (SMA)) via a frontal light diffuser providing a circular spot directly in contact with the skin (illuminated surface: $\approx$ 5mm$^2$). 1 ms separated each pulse, so that the system would have time to desexcite fully between each pulse.

The fluorescence ($\approx$ 10 ns) and delayed fluorescence (5 to 500 $\mu s$) were collected by a fiber next to the light diffuser, and subsequently sent to a Photomultiplier (PMT) (Hamamatsu H11526-20- NF) and amplifier (Hamamatsu C6438-01). The PMT was gated to reject the prompt fluorescence. The decays were recorded by a data acquisition board (DAQ NI USB 6259, National Instruments) and processing unit. The emission light was filtered using a band-pass filter in the [600; 700] nm region, to reject excitation light and keep only the DF of PpIX. For certain measurements, the DF signal was additionally filtered to extract specific spectral bands, using bandpass filters at 625 $\pm$ 12 nm, 670 $\pm$ 12 *nm* or 700 $\pm$ 12 *nm.* OD outside of bandpass: > 4. When needed, the excitation intensity was varied by placing neutral density filters (OD $\in$ [0.15; 0.9]) in the excitation beam. In this work, DF energy $E_{DF}$ is understood as the total energy of the DF: if *I(t)* is the DF intensity as a function of time as measured by the data acquisition, then the intensity of the DF is $E_{DF} = \int_0^{+\infty} I_{DF}(t)\, dt.$ When changing the excitation intensity, the initial concentration of $T_1$ states is linearly changed, as all processes leading to its formation are described by linear Einstein equation (absorption and Intersystem Crossing), and the illumination zone is constant in size. In short, $[T_1]_0 \propto I_{ex}$. As a consequence, when $[T_1]_0$ changes, $I_{DF}$ is likely to vary linearly if the DF mechanism involves one $T_1$ molecule, or quadratically, if it involves two $T_1$ molecules. Unless otherwise mentioned, the DF intensity was manually fitted to a decreasing mono-exponential $I_{DF}(t) = I_0 e^{-t/\tau} + y_0$ using the built-in fitting algorithm of the Origin V8 software.

### Results

Contrary to the *in vitro* situation, PpIX concentration is not known *in vivo,* which makes the analysis more complex: we are unable to compare the DF at different concentrations, we have to track other proofs of the existence of excimers. The inventors chose to investigate the competition process that occurs between the 630 and 670nm luminescence pathways.

### Evolution of $E_{670}/E_{630}$ compared to $\tau_{630}$

To assess the existence of excimers, the inventors looked at the DF energy at 670 nm. But the monomer form also has a slight DF signal at this wavelength (Fig. 3). We therefore compute the ratio of energy at 670 and 630 nm, $E_{670}/E_{630}$.

The inventors could have chosen to study the ratio of initial intensities, because it would not depend on the quenching by oxygen, which can be slightly varying between the measurement at 670 and 630 nm. But, in reality, the initial intensity is highly dependent on the second-order mechanisms described rigorously below, which do not follow the Stern Volmer equation. The inventors thus concluded that the DF energy (time integral of the intensity) is more robust for such an analysis.

If excimers did not exist, the ratio of energy at 670 and 630 nm would be constant over time. Showing its variations could be enough a proof. But to make it more compelling, the inventors compared the possible variations of this ratio with the DF lifetime at 630 nm $\tau_{630}$: if excimers exist, these two variables should be anti-correlated: When the PpIX concentration is high, $\tau_{630}$ small. At the same time, excimers form and produce DF at 670 nm, which causes $E_{670}/E_{630}$ to be high. Conversely, when the PpIX concentration is reduced, $\tau_{630}$ is longer, and no excimers form so that $E_{670}/E_{630}$ is small.

The result is shown in Fig. 8. Averaging the variations for a large number of patients would erase the erratic changes due to the local PpIX inhomogeneities and would only reflect the average bulk PpIX concentration (thus repeating Fig. 2). To detect the effect of local excimer formation, one has to compare $\tau_{630}$ and $E_{670}/E_{630}$ for each patient.

Nevertheless, to prove that this phenomenon was common in our measurements, we add the corresponding graphs of 4 other volunteers on Fig. 9. The inventors try to confirm their finding by comparing the DF lifetime at 630 and 670 nm in two different conditions. First, they apply a standard method of compression of the probe against the skin for one minute before measuring. As documented, the pressure blocks the microcirculation, and soon the quasi-totality of the intracellular oxygen is consumed, bringing the $pO_2$ close to 0. At this point, the quenching by oxygen is removed, and the competition between the monomer and excimer becomes clearly visible. In such case, $\tau_{630}$ and $\tau_{670}$ should, once again, be anticorrelated over time.

The result is presented in Fig. 10. In the case where the microcirculation was not compressed, and $pO_2$ is around physiologic values, a different behaviour is found: since the quenching by oxygen is very strong, it defines the lifetime, and the competition between monomer and excimer form becomes secondary. The inventors found $\tau_{630}$ and $\tau_{670}$ to be correlated, as they are defined by the oxygen quenching. Fig. 15 shows the results. These measurements all speak in favour of the existence of excimers *in vivo*. The inconsistencies detailed above could potentially have found an answer. However, the fact that excimer formation, a second-order process, plays a significant role *in vivo* in the tested conditions, opens an unforeseen question: is it sure that P-type DF, a second order DF process ($2T_1 \rightarrow S_1 + S_0$), does not occur? Could one bimolecular process appear and another one, starting with the same reactants, be negligible?

### *Contribution of P-type DF*

The easiest method to discriminate between E and P-type DF is to study the dependence of DF energy on excitation intensity: in the case of a monomolecular process (E-type), this dependency should be linear, while in the case of a bimolecular one (P-type), it should be quadratic. Indeed, the initial concentration of triplet state $[T_1]_0$ is proportional to the excitation intensity, as the illuminated volume is constant. By fitting a second order polynome

$$E_{DF} = aI_{ex}^2 + bI_{ex} + c$$

one can compare the linear contribution $bI_{ex}$ to the quadratic one $aI^2_{ex}$. An example of such curve is presented in Fig. 11. This type of measurement was repeated several times to confirm its conclusions, in various conditions (compressed or not, different times of ALA application). In addition to the Dumbo setup (described above), the same experiment was conducted on the "Opotek" setup, described in Harms et al., 2013. The radiant exposure in the Dumbo setup is 300 $\mu J.cm^{-2}$ per pulse, which is comparable to the Opotek (10 to 100 $\mu J.cm^{-2}$ per pulse). The averaged quadratic contributions are presented in table 1.

Table 1: P-type contribution in the total DF signal, at different ALA application time and $pO_2$ levels, on two distinct setups

| Time of ALA application | Dumbo | Dumbo - $pO_2$ = 0 | Opotek |
|---|---|---|---|
| 3h | / | 30% | / |
| 5h | 70% | 40% | 40% |
| 7h | 70% | 40% | 40% |

Several conclusions can be drawn from these experiments:

- In standard, uncompressed measurements, P-type DF contributes between 40% to 70% to the total DF signal.

- The absence of oxygen significantly reduces the contribution of P-type DF. The increase of the contribution of E-type DF can be understood as a kinetic competition between first order processes, E-type DF and oxygen quenching: $T_1 \rightarrow S_1$ versus $T_1 + {}^3 O_2 \rightarrow S_0 + {}^1 O_2$.
- Consequently, it is preferable not to neglect the contribution of P-type DF in the analysis of DF signals, as has been done so far. In the next section, the inventors present a precise model to take different DF processes into account.

### *Influence of second order reactions on DF processes: the kinetics of non-monoexponential decays*

### *Evolution of the triplet state: the depopulation equation*

### Reminder on kinetics

Considering the reaction $A \xrightarrow{k} B$ we define the reaction rate constant:

$$\xi(t) = -\frac{\partial A}{\partial t} = \frac{\partial B}{\partial t} \tag{5}$$

under the hypothesis of the law of mass action (reaction rate proportional to the product of the concentration of all reactants at the power of their stoichiometric coefficients), the reaction rate is

$$r = k[A] \tag{6}$$

with $k$ the reaction rate constant, which, as a first approximation, depends minimally on concentrations of reactants. In our case of excited states dynamics, the situation is identical: reaction rates are also proportional to the reactants concentration, following the linear Einstein equations. When a reactant appears with a coefficient 1, in the equation, its concentration can be computed by solving the linear differential equation:

$$\frac{\partial A}{\partial t} = -k[A] \tag{7}$$

The result is an exponential function:

$$[A](t) = [A]_0 e^{-kt} = [A]_0 e^{-t/\tau} \tag{8}$$

which allows the introduction of a characteristic time $\tau = 1/k$, which does not depend on *[A]*. It is called the lifetime of A. To be completely clear, **the population evolves as a mono-exponential if and only if it is only depopulated by first-order reactions.** A mono-exponential kinetics is equivalent to a first-order depopulation. But as soon as two or more coefficients appears in front of A, we are in the non-linear case:

$$2A \xrightarrow{k'} B \tag{9}$$

This case follows the kinetics:

$$\frac{\partial A}{\partial t} = -2k'[A]^2 \tag{10}$$

where the concentrations evolution is harder to determine, and is not mono-exponential. When a reactant appears with a coefficient one, we will indifferently designate the process as linear, monomolecular or first-order. Conversely, when the stoichiometric coefficient is 2, we use the terms non linear, bimolecular or second-order.

**The traditional Stern-Volmer model**

In the traditional model, the assumption is that only E-type DF, Internal Conversion and oxygen quenching contribute to the triplet state desexcitation:

$$\frac{\partial[T_1]}{\partial t} = -(k_E + k_{IC} + k_q^{O_2}pO_2)[T_1] \qquad (11)$$

**Taking second-order desexcitation in account**

The inventors have shown above that the contribution of P-type DF was not negligible. They have also identified a potentially strong effect of excimer formation, which is also bimolecular. Based on this changed technical understanding of the processes involved, they include the quadratic desexcitation terms in the depopulation equation, which thus loses its linear nature (see definition of reaction rates on Fig. 7:

$$\frac{\partial[T_1]}{\partial t} = -\left(k_E + k_{IC} + k_q^{O_2}pO_2\right)[T_1] - 2(k_P + k^d)[T_1]^2 \qquad (14)$$

This solves into a more complex function. As it involves both first and second-order reactions, it is designated the mixed-orders depopulation function:

$$[T_1]_{mixed}(t) = \frac{k_E + k_{IC} + k_q^{O_2}pO_2}{(\frac{k_E + k_{IC} + k_q^{O_2}pO_2}{[T_1]_0} + 2(k_P + k^d))e^{\left(k_E + k_{IC} + k_q^{O_2}pO_2\right)t} - 2(k_P + k^d)} \qquad (15)$$

In this expression, one can spot the exponential term $e^{-\left(k_E + k_{IC} + k_q^{O_2}pO_2\right)t}$, perturbed by the second-order constants $k_P + k^d$. We can study approximate forms of the function at short and long times:

**Mixed-orders depopulation at short times**

When t→0

$$[T_1]_{mixed}(t)\underset{0}{\sim} \frac{[T_1]_0}{1 + (k_E + k_{IC} + k_q^{O_2}pO_2 + 2(k_P + k^d)[T_1]_0)t} \qquad (16)$$

which yields, [Ti](0) = $[T_1]_0$, but also $\frac{\partial[T_1]}{\partial t}(t = 0) = -(k_E + k_{IC} + k_q^{O_2}pO_2 + 2(k_P + k^d)[T_1]_0)[T_1]_0$ And if we assume that $[T_1]_0$ is big enough, this even reduces to

$$[T_1]_{mixed}(t)\underset{0}{\sim} \frac{[T_1]_0}{1 + 2(k_P + k^d)[T_1]_0 t} \text{ and } \frac{\partial[T_1]}{\partial t}(t = 0) = -2(k_P + k^d)[T_1]_0^2 \qquad (17)$$

Thus, the beginning of the depopulation is completely controlled by the second-order processes, and its slope reflects the reaction rate constants $k_p$ and $k^d$ and the initially excited population $[T_1]_0$.

**Mixed-orders depopulation at long times**

When t → +∞

$$[T_1]_{mixed}(t) \underset{+\infty}{\sim} \frac{(k_E + k_{IC} + k_q^{O_2} pO_2)[T_1]_0}{k_E + k_{IC} + k_q^{O_2} pO_2 + 2(k_P + k^d)[T_1]_0} e^{-\left(k_E + k_{IC} + k_q^{O_2} pO_2\right)t} \quad (18)$$

a mono-exponential decay of lifetime follows. exactly like the Stern-Volmer theory:

$$\tau_\infty = \frac{1}{k_E + k_{IC} + k_q^{O_2} pO_2}$$

This shows that the later part of the desexcitation follows a Stern-Volmer quenching by oxygen.

However, the DF signal $I_{DF}(t)$ is only an indirect image of the triplet population $[T_1](t)$: to draw conclusions on how to analyse the decays, the inventors investigated this link.

**From triplet concentration evolution to DF signals**

The DF intensity produced by E-type DF is worth:

$$I_{DF}^E(t) = \theta_{S_1} k_E [T_1](t) \qquad (19)$$

with $\theta_{S_1} = \frac{k_f^S}{k_f^S + k_{nr}^S + k_{ISC}}$ the fluorescence quantum yield of the $S_1$ state. Conversely the DF intensity produced by P-type DF is:

$$I_{DF}^P(t) = \theta_{S_1} k_P [T_1]^2(t) \qquad (20)$$

and finally the excimer DF intensity:

$$I_{DF}^{excimer}(t) = \theta_{D^*} k^d [T_1]^2(t) \qquad (21)$$

with $\theta_{D^*} = \frac{k_f^d}{k_f^d + k_{nr}^d}$. the quantum yield of the excimer state. The expected DF intensity can be deduced by injecting the expression of [T1] of equation (15) into equations (19) to (21).

**Pure second-order desexcitation and DF**

The inventors have further explored the result of a process where second-order reactions are so much stronger than first order ones that the depopulation equation becomes:

$$\frac{\partial [T_1]}{\partial t} \approx -2(k_P + k^d)[T_1]^2 \qquad (22)$$

In that case, the solution is

$$[T_1](t) = \frac{[T_1]_0}{1 + 2(k_P + k^d)[T_1]_0 t} \qquad (23)$$

and finally, it is logical to consider that only P-type will produce DF:

$$I_{DF}^{P}(t) = \theta_{S_1} k_P \left(\frac{[T_1]_0}{1 + 2(k_P + k^d)[T_1]_0 t}\right)^2 \qquad (24)$$

To assess the existence of P-type or excimer DF *in vivo,* the inventors compared the quality of a mono-exponential fit versus a mixed-orders decay fit on the DF signals collected. This is done in the next section.

**Results**

The inventors assessed the nature of the DF process *in vivo,* at standard excitation intensity. The central hypothesis is that the depopulation equation is of mixed orders, and the DF is caused by a mix of E-type and P-type. Thus, the inventors' proposition for the most exact fit is:

$$I_{DF}^{630}(t) = \alpha I_{DF}^{E} + \beta I_{DF}^{P} = \alpha \theta_{S_1} k_E [T_1](t) + \beta \theta_{S_1} k_P [T_1]^2(t) \qquad (25)$$

with *[T₁](t)* from equation (15). Unfortunately, this function requires sophisticated fitting methods.

To allow for fitting with more standard type fitting methods, e.g., as implemented in Origin, the inventors compared the fitting quality of 4 distinct functions: Mono-exponential (1st order depopulation and E-type DF), Mixed-orders + E-type DF, mixed-orders + P-type DF, and 2nd order + P-type DF (equation (24)). In essence, the inventors are assessing the nature of two reactions:

- The reaction that will depopulate the triplet, whose kinetics will define the depopulation equation. The inventors call it the **depopulation pathway.** This reaction can be of the first order (Internal Conversion, quenching by oxygen), or of the second order (P-type RISC).

- The reaction that will produce the delayed fluorescence. The inventors call it the **measurement pathway.** In our case, it can be E-type DF or P-type DF.

The combination of these two reactions defines the expected kinetics of the DF. The different possibilities and associated kinetics are summarized in Table 2.

| Row: measurement pathway Column: depopulation pathway | E-type DF | P-type DF |
|---|---|---|
| 1st order (ex: IC) $$\frac{\partial [T_1]}{\partial t} = -a[T_1]$$ | $S_{DF}(t) = \theta_{S_1} k_E [T_1]_0 e^{-at}$ | $S_{DF}(t) = \theta_{S_1} k_P [T_1]_0^2 e^{-2at}$ |
| 2nd order (ex: P-type) $$\frac{\partial [T_1]}{\partial t} = -b[T_1]^2$$ | $$S_{DF}(t) = \theta_{S_1} k_E \frac{[T_1]_0}{1 + b[T_1]_0 t}$$ | $$S_{DF}(t) = \theta_{S_1} k_P \left(\frac{[T_1]_0}{1 + b[T_1]_0 t}\right)^2$$ |
| Mixed-orders $$\frac{\partial [T_1]}{\partial t} = -a[T_1] - b[T_1]^2$$ | $S_{DF}(t) = \theta_{S_1} k_E [T_1]_{mixed}(t)$ | $$S_{DF}(t) = \theta_{S_1} k_P [T_1]_{mixed}^2$$ |

Table 2: Delayed fluorescence kinetics for different depopulation and measurement pathways. Notation:

$$a = k_E + k_{IC} + k_q^{O_2} pO_2, \ b = 2(k_P + k^d)$$ and $[T_1]_{mixed}$ : expression from equation 15. 1st order depopulation and P-type DF is unlikely to happen in reality, because 1st order is associated to low concentration of excited states, while P-type corresponds to high concentrations. The same remark goes for 2nd order depopulation and E-type DF.

To determine which reactions are the measurement and depopulation pathways, the inventors consider standard 630 nm DF signals obtained on volunteers' sternum after 3 to 7 hours of ALA application. The inventors compared the statistical quality of different fits in Table 3.

These measurements further confirm that P-type and excimer DF are so strong that they significantly change the shape of the decays. Moreover, the great success of second-order + P-type fitting function (introduced above) suggests that most triplet state molecules desexcite via P-type RISC and DF. This does not mean that oxygen quenching is negligible: as described in equation 18, it controls the slope of the decay at longer times. Even if it has a moderate influence on the whole decay (and thus the global shape mainly depends on second-order processes), it has a strong influence on the lifetime that one can extract from the signal. It should also be considered that multi-parameters non-linear fittings are challenging: it is possible that, mixed-orders + P-type DF should the best fit.

**Conclusion**

In this section, the inventors have shown the necessity to take second order processes into account in order to correctly interpret the DF kinetics, and have exhibited the appropriate decay function. This allows for the development of methods that take these effects

Table 3 : Fitting parameters and precision of different fitting functions. Notations defined in table 2. The $2^{nd}$ order + P-type DF function is the most precise. Different methods indicate the second-order rate constant, $b = 2(k_P + k^d) = 10^8 \, M^{-1}s^{-1}$, and the initial concentration of excited triplet states $[T_1]_0 \in [28; 51] \, \mu M$. This high value is another element which suggests that intracellular PpIX concentration could be higher than previously thought.

| Fitting function | $r^2$ | $\alpha \, [s^{-1}]$ | $b \, [M^{-1}S^{-1}]$ | $[T_1]_0 \, [M]$ |
|---|---|---|---|---|
| Mono-exp | 0.913 | $1.15 \times 10^4$ | $l$ | $l$ |
| Mixed-orders + E-type | 0.936 | $8.46 \times 10^{-10}$ | $1.14 \times 10^9$ | $2.82 \times 10^{-5}$ |
| Mixed-orders + P-type | 0.933 | $2.73 \times 10^{-8}$ | $2.04 \times 10^8$ | $4.79 \times 10^{-5}$ |
| $2^{nd}$ order + P-type | 0.996 | $l$ | $2.5 \times 10^8$ | $5.1 \times 10^{-5}$ |

into account and measure the $pO_2$ more precisely. The correction can be done at different levels of accuracy and practical feasibility, described below.

***Practical consequences on skin mitochondrial oxygen measurement***

Beside the new understanding of PpIX DF, the objective of the invention remains to improve the quality of cellular oxygen measurements, in particular, in patients. More specifically, the objective is to solve the inconsistencies described above. The inventors describe several methods in this section that solve this problem.

**1. The decay central fitting (DCF) method to capture the linear quenching processes**

As detailed above, a mixed-order decay (equation (15)) can be temporally subdivided in three distinctive parts:

- The beginning of the decay is not mono-exponential, and governed by a mix of first and second order processes (equation (17)). Its expression involves $[T_1]_0$, which means that it depends on excitation intensity and local chromophore concentration. One simple way to obtain a stable $pO_2$ value thus is not to fit this part of the decay which is completely relative and changing.

- The central part of the decay is a mono-exponential whose inverse lifetime is directly proportional to the $pO_2$ (equation (18)). Its lifetime does not depend on $[T_1]_0$. Even if the amplitude depends on the local fluence rate and chromophore concentration, the lifetime (i.e. the slope of the decay) does not: this section of the decay may be fitted by a mono-exponential.

- The last part of the decay is dominated by noise. It is preferably neglected and not fitted.

This subdivision is illustrated in Fig. 12. This splitting shows that only the center of the decay should be fitted to a mono-exponential, and this is enough to precisely deduce the $pO_2$, despite the second-order effects. The inventors call this approach the decay central fitting method. It is not an experimental optimization of the fitting quality: it reflects an improved conceptual understanding of the luminescence process.

The determination of $t_1$, the time of transition between the initial non-exponential phase and the monoexponential phase, and $t_2$, the time between the monoexponential phase and the noise-dominated section, works as follows: assuming that the DF intensity $I(t)$ can be written as the sum of noise $\Delta I(t)$, centered around 0, and an average intensity $\bar{I}(t)$, which is typically the function obtained after fitting of the experimental signal, such that $I(t) = \bar{I}(t) + \Delta I(t)$.

Defining the monoexponentiality indicator as

$$M(t) = (1 - \frac{\bar{I}'' \bar{I}}{(\bar{I}')^2})^2$$

With ' denoting the derivation with respect to time. The thresholds are values such that: $M(t_1) = 0.7 = T$.

Introducing the signal to noise ration SNR(t), the noise dominated section starts, for example, at the time value such that

$$SNR(t_2) = \frac{\bar{I}(t_2) + \Delta I(t_2)}{\bar{I}(t_2)} = 2 = T'$$

The inventors set the optimal values for the decay central fitting method to $[t_1; t_2] = [275; 600]$ $\mu s$ in the experimental conditions used (i.e., in the Dumbo setup) based on the criteria and considerations explained above.

A last point should be discussed: the lifetime indicated by the plateau (long $225\mu s$ in this experiment on non-compressed skin),

$$\tau_T = \frac{1}{k_E + k_{IC} + k_q^{O_2} pO_2}.$$

is in reality not the triplet lifetime  When produced by E-type DF, the DF intensity in the central range is indeed proportional to $e^{-\frac{t}{\tau}}$, but when produced by P-type DF, which represents 40 to 70% of the signal, the decay lifetime is worth half of the triplet lifetime due to the quadratic dependence. Consequently, the observed lifetime is an average between $\tau_T$ and $\tau_T/2$. The weights of this average cannot be simply measured by this method, and depends on the local PpIX concentration and fluence rate. The variable excitation fluence rate can be used to determine the relative contributions of E-type and P-type DF, and thus calculate $\tau_T$.

To assess the efficiency of this method, the data of Fig. 2 was analysed on the reduced interval [275; 600] $\mu s$. The result is presented in Fig. 14.

The lifetime calculated is less erratic than before application of the DCF method, but some variability remains as $\tau$ varies from 100 to 350 $\mu s$. This could be due to the variable contribution of E and P-type giving a random fluctuation between $\tau_T$ and $\tau_T/2$. It suggests that the DCF method can still be rendered more precise with a quantitative discrimination between mono and bimolecular processes.

**Conclusion**

The decay central fitting method constitutes a step-forward towards a stable, precise measurement of the $pO_2$. It is easy to apply, as it only requires to place a 630 nm bandpass filter in the emission beam and to change the mono-exponential fitting range to the time between $[t_1; t_2]$, e.g., [275; 600] $\mu s$.

As its precision is limited by the unknown E-type vs P-type DF ratio, it is preferred to simultaneously vary the excitation intensity, quantify the fraction of linear vs quadratic behaviour (as done in the section "contribution of P-type DF" above), and subsequently deduce a more precise $\tau_T$ and $pO_2$.

**2. Correction of PpIX self-quenching: the adaptive Stern-Volmer relationship**

Another option, which would be conceptually less different from the standard model, but practically more complex, is to conserve the Stern-Volmer model, but to adapt the DF lifetime in absence of oxygen at 630 nm $\tau_0^{630}$, in order to compensate for the variable self-quenching of PpIX. Keeping in mind the central results discovered by the inventors: first, that PpIX lifetime in absence of oxygen strongly depends on the local PpIX concentration, in a Stern-Volmer like quenching relationship, and second, that the ratio of DF energy at 670 and 630 nm is linearly linked to the local PpIX concentration. The method comprises:

- measuring the DF signal at 630 nm and 670 nm simultaneously and in the same sample volume,

- using the ratio of energies $\dfrac{E_{670}}{E_{630}}$ to deduce the local concentration of PpIX,

- deduce $\tau_0{}^{630}$ out of [PpIX] according to the Stern-Volmer quenching relationship established earlier.

- injecting $\tau_0{}^{630}$ and $\tau^{630}$ into the adaptative Stern-Volmer relationship, and finally calculating the corrected $pO_2$:

$$pO_2^{corrected} = \frac{1}{k_q^{O_2}}\left(\frac{1}{\tau_{630}} - \frac{1}{\tau_0^{630}}\right) \qquad (26)$$

As shown in Fig. 22, it has been proven *in vitro* that the energies (time integrals if the decays) at 670nm and 630nm were linearly correlated to the concentration of PpIX.

An application of this method is provided in Fig. 16. The graph shows a much more constant value for the concentration of oxygen, and thus, a significantly better assessment of the status of the subject.

**Conclusion**

This method has shown very good results: on volunteers whose $pO_2$ had no reason to vary abruptly, the adaptive Stern-Volmer relationship flattened the $pO_2$ around a stable value, which is physiologically more realistic. The method was very robust and yielded good results even on noisy data.

In terms of practical modifications, the method requires the selection of 2 emission wavelengths or spectral bands at about 630 and about 670 nm, thus, e.g., bandpass filters may be installed. The algorithmic part remains simple, as the fitting function is a mono-exponential. The inventors present the adaptative Stern-Volmer relationship as a most satisfactory method, in terms of precision and ease of implementation.

**3. The complete method: fitting mixed-orders decays**

As detailed above, when taking into account all different reactions, the DF intensity at 630 nm follows:

$$I_{630}(t) = \theta_{S_1}\left(k_E[T_1]_{mixed} + k_P[T_1]_{mixed}{}^2\right) + y_0 \qquad (27)$$

with

$$[T_1]_{mixed}(t) = \frac{k_E + k_{IC} + k_q^{O_2}pO_2}{\left(\dfrac{k_E + k_{IC} + k_q^{O_2}pO_2}{[T_1]_0} + 2(k_P + k^d)\right)e^{\left(k_E+k_{IC}+k_q^{O_2}pO_2\right)t} - 2(k_P + k^d)} \qquad (28)$$

*using notations introduced earlier.*

The most precise method would be to fit this function on each decay. Once the different fixed parameters have been determined ($\theta_{S_1}$, $k_E$, $k_P$, $k_{IC}$, $k_q^{O_2}$, $k^d$), only two variables remain to be fitted: $[T_1]_0$ and $pO_2$.

To reinforce the result, it is also possible to fit the decay at 670 nm, which is worth

$$I_{DF}^{670} = \theta_{D^*}k^d[T_1]^2(t) \qquad (29)$$

Also, once typical values of $[T_1]_0$ and $pO_2$ are known under the experimental conditions used, it is possible to start the optimization algorithm close to these values. A tailor-made algorithm can then be provided that reaches optimal parameters in a short time.

The application of this method in practice is complex: fitting of such a complex nonlinear function is a numerical challenge.

### *Exclusion of an in vivo contribution of Singlet Oxygen Feedback Delayed Fluorescence (SOFDF)*

An additional mechanism of PpIX-DF has been recently described in artificial systems, e.g., by Vinklarek et al., 2017. It involves a double action of oxygen: a ground state triplet molecule collects the PpIX triplet energy, as in conventional quenching, but then this singlet oxygen excites another $T_1$ molecule to $S_1$, which subsequently produces SOFDF. The process can be summed up by the following reaction:

$$SOFDF: \begin{cases} T_1 + {}^3O_2 \rightarrow S_0 + {}^1O_2 \\ T_1 + {}^1O_2 \rightarrow S_1 + {}^3O_2 \\ \qquad S_1 \overset{h\nu}{\rightarrow} S_0 \end{cases} \qquad (30)$$

This process has been observed *in vitro* (Vinklarek et al., 2017; Scholz et al., 2013. Photochemical & Photobiological Sciences 12: 1873) and in isolated fibroblasts (Scholz et al., 2017. Photochemical & Photobiological Sciences 16: 1643-1653; Scholz et al., 2015. Photochemical & Photobiological Sciences 14: 700-713), with different photosensitizers, including PpIX. This type of DF is characterized by a rise-decay kinetics, described by

$$I_{SOFDF}(t) = C(e^{-t/\tau_1} - e^{-t/\tau_2}) \qquad (31)$$

with $\tau = \dfrac{\tau_T}{1 + \tau_T/\tau_\Delta}$ the decay time and $\tau_2 = \tau_T/2$ the rise time ($\tau_T$: triplet state lifetime and $\tau\Delta$ : singlet oxygen lifetime).

Despite careful observations, the inventors were not able to identify the rise decay kinetics *in vivo,* suggesting that SOFDF did not significantly occur in human skin. This can be explained by two reasons:

* First and foremost, all SOFDF observations with cells in the state of the art were done on air saturated samples of isolated cells, where the $pO_2$ must have been close to 160 *mm Hg.* In tissues such as human skin, the $pO_2$ is closer to 50 *mm Hg,* i.e, at least three times smaller. Since SOFDF involves two oxygen molecules, it requires a high $pO_2$ to be detectable. In the tissues and organs analysed in the context of the invention, the $pO_2$ is too low to detect SOFDF.

* The spatial irradiance used by Vinklarek et al., 2017, was around 230 times more intense than what is used in the Dumbo setup (70 $mJ.cm^{-2}$ vs 300 $\mu J.cm^{-2}$). This could explain a different behaviour.

### *Compatibility with previous measurements (oxygen consumption)*

A natural question after the discovery of PpIX excimers is: how measurements and conclusions in the past have been performed, using an incomplete model of triplet-state based optical properties, such as delayed fluorescence of probes, e.g., PpIX? The case of Oxygen Consumption Measurements (OCM) is typical: as described in the literature described above, this method, based on the compression of the microcirculation to assess the rate of oxygen consumption by skin cells, has shown excellent results in recent years. How can the results have been so coherent, if the fitting method was incorrect? To evaluate it, the inventors compared the $pO_2$ consumption curve obtained 1) in the physiologic case, according to the models of oxygen consumption as provided by the present invention, and 2) if this physiologic $pO_2$ was deduced using the standard Stern-Volmer method, while in reality the PpIX decay follows the mixed-orders + P-type function (equation (15)):

According to simple models of oxygen consumption by tissue, in case of microcirculation blockage by compression, the $pO_2$ evolution can be simplified to:

$$pO_2^{real}(t) = 1 + e^{-t} \qquad (33)$$

where we arbitrarily set the time constant to 1. Assuming that the $pO_2$ varies as $pO_2{}^{real}$, the DF signal that would be obtained if the DF process was a mixed-orders + P-type DF (equation (15)) was deduce, as suggested by table 3. This gives us a series of signals $DF^{real}(t)$ for each value of $pO_2{}^{real}(t)$. Then mono-exponential decays were incorrectly fitted to the different $DF^{real}(t)$, thus obtaining an incorrect $pO_2^{incorrect}(t)$ thanks to the standard Stern-Volmer relationship. Finally, $pO_2^{real}$ was compared to $pO_2^{incorrect}(t)$ to see how different the $pO_2$ becomes when one uses an incomplete model of PpIX DF.

The result is presented in Fig. 17. This graph shows important information: even when using an improper Stern-Volmer model of PpIX DF, the $pO_2$ obtained is not too different from the reality, for the simple reason that whatever the model, when the $pO_2$ increases, the DF characteristic time decreases, and vice-versa.

As a conclusion, it is perfectly possible that a large number of studies were conducted based on an improper model of PpIX model, giving sometimes satisfactory results: it is only when looking in detail into the DF shape and lifetime variations that the inconsistencies can be detected, and then overcome by the method of the invention.

**Claims**

1. A method for determining the concentration of a quencher, wherein the quencher preferably is oxygen, and/or the concentration of a probe capable of exhibiting a triplet-state based luminescence or transient triplet absorption in a cell in a tissue or organ, comprising steps of

   a) exciting the probe by irradiation with light having an wavelength and a fluence rate suitable for excitation of the probe,

   b) measuring the temporal evolution of the triplet-state based luminescence or transient triplet absorption of the probe at at least one emission or absorption wavelength, wherein, in the presence of the quencher, the triplet-state luminescence or transient triplet absorption decays more quickly compared to the decay in the absence of the quencher, and

   c) correlating said temporal evolution with said concentration(s),

   wherein second order reactions involving two excited triplet states of the probe are considered,

   wherein, when the quencher is oxygen, the probe optionally is a heme precursor such as protoporphyrin IX (PpIX).

2. A method of claim 1, wherein the temporal evolution of triplet-state based luminescence selected from the group consisting of delayed fluorescence or phosphorescence is measured, preferably, the temporal evolution of delayed fluorescence,

   wherein, optionally, the quencher is oxygen and the probe is a heme precursor such as PpIX.

3. The method of any of claims 1 or 2, wherein the concentration of excited triplet states of the probe is considered for determining the concentration of the quencher or of the probe,

   wherein, preferably, the concentration of the probe is determined, and, optionally, then, the concentration of the quencher is determined.

4. The method of any of the claims 2 or 3, wherein the emission of the delayed fluorescence is measured simultaneously at at least two wavelengths, in the same sample volume, wherein these two wavelengths are suitable for distinguishing delayed fluorescence of probe monomers and of probe excimers,

   wherein, preferably, the probe is a heme precursor such as PpIX and the first wavelength is in the range of 615-645 nm, optionally, about 630 nm and the second wavelength is in the range of 646 nm-700 nm, optionally, about 670 nm.

5. The method of claim 4, wherein the adaptive Stern-Volmer relationship is used for determining the concentration of the quencher, wherein the adaptive Stern-Volmer relationship

   i) calculates the concentration of the probe based on the ratio of the probe excimer DF energy or initial intensity to the probe monomer DF energy or initial intensity

   ii) calculates the delayed fluorescence lifetime in absence of quencher at the probe concentration calculated in step i), and

   iii) calculates the quencher concentration by feeding the DF lifetime in absence of oxygen calculated at step ii) into the Stern-Volmer relationship, thus adapting the Stern-Volmer relationship to the probe concentration,

   wherein, preferably, the probe is a heme precursor such as PpIX and the ratio $E_{670}/E_{630}$ is used in step i).

6. The method of any of the preceding claims, wherein the fluence rate (intensity) or fluence of the light for excitation is used to determine the relative contribution of first and second order triplet deactivation and / or luminescence processes

   wherein, preferably, the probe is excited by irradiation with light having at least two, optionally, five different fluence rates or fluences.

7. The method of any of claims 1-2, wherein the decay central fitting method is used for correlating said temporal evolution with said concentration of quencher, wherein the decay central fitting method rejects the initial non-exponential part of the decay and, optionally, rejects the tail of the decay dominated by noise, thus selecting the central part of the decay of delayed fluorescence intensity over time.

8. The method of any of the preceding claims, wherein the concentration of the quencher is determined on the basis of the assumption that the excited triplet state of the probe deactivates through a mix of first and second order reactions and/or on the assumption that the delayed fluorescence is caused by a mix of first and second order reactions.

9. The method of any of the preceding claims, wherein the probe is a heme precursor such as PpIX and the quencher is oxygen, and, before step a), a precursor of PpIX, optionally, 5-aminolevulinic acid, is administered to the cell or has been administered to the cell.

10. The method of any of the preceding claims, wherein the cell is part of a tissue or organ, wherein, preferably, the cell is *in situ* in a patient, wherein, optionally, the tissue is skin.

11. A method for assessment of mitochondrial function in a sample comprising a tissue or organ, comprising restricting or ceasing the supply of oxygen to said sample and carrying out the method of any of the preceding claims with said sample, wherein the probe is PpIX and the quencher is oxygen.

12. A method for assessment of the status of a patient selected from the group comprising a sepsis patient, a critically ill patient, a patient undergoing a tumor treatment (e.g., phototherapy or photodynamic therapy), a patient undergoing surgery, a patient suffering from a neurodegenerative condition, such as Alzheimer, Parkinson or Huntington diseases, or a decubitus patient, or for selecting an organ potentially suitable for transplantation to a patient, comprising carrying out the method of any of the preceding claims, wherein the cell is a cell of said patient or organ, optionally, wherein the cell is *in situ* in a patient or organ.

13. A precursor of protoporphyrin IX, preferably, 5-aminolevulinic acid, for use in an *in vivo* method of any of the preceding claims, wherein the probe is a heme precursor such as PpIX and the quencher is oxygen and the cell is in a human or animal subject.

14. A device suitable for carrying out the method of any of claims 1-13, comprising

- an excitation light source arranged to illuminate a sample volume, and
- a time and intensity-resolved light detector arranged to detect luminescence, such as fluorescence or phosphorescence from the sample volume, or triplet triplet absorption by the sample volume, and
- a control unit configured to obtain measurements of luminescence, such as fluorescence or phosphorescence, from or triplet triplet absorption by the sample volume,
wherein said device comprises

i) means for determining the delayed fluorescence lifetimes and intensities of a probe simultaneously at two emission wavelengths suitable for distinguishing delayed fluorescence of probe monomers and of probe excimers, wherein, preferably, the probe is a heme precursor such as PpIX, delayed fluorescence is determined, and the first wavelength is in the range of 615-645 nm, optionally, about 630 nm and the second wavelength is in the range of 646 nm-700 nm, optionally, about 670 nm; and/or
ii) means for exciting the probe with at least two, optionally, five different excitation fluence rates or fluence; and/or

wherein the device optionally further comprises iii) means for exciting the probe with at least two, optionally, five different excitation wavelengths.

15. The device of claim 14, further comprising

• a pressure pad for applying local pressure on tissue containing arterioles, veins and/or capillary bed that supply oxygen to the sample volume, and/or

• a processing unit capable of processing the obtained measurements and configured to apply the adaptive Stern-Volmer relationship and/or the decay central fitting method and/or the mixed orders fitting method, preferably, at least the adaptive Stern-Volmer relationship.

16. Use of the device of any of claims 14-15 for determining the concentration of a quencher such as oxygen and/or of a probe such as PpIX in a cell according to the method of any of claims 1-12.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

Fig. 17

Europäisches
Patentamt

European
Patent Office

Office européen
des brevets

# EUROPEAN SEARCH REPORT

Application Number

EP 20 17 6067

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 1 742 038 A1 (AMC AMSTERDAM [NL]) 10 January 2007 (2007-01-10) | 1-3, 9-13,16 | INV. G01N21/64 |
| Y | * paragraphs [0007] - [0054], [0060] - [0071] * <br> * figures 1, 3-10, 12, 13 * | 4-8,14, 15 | |
| Y | EP 2 728 344 A1 (KYOTO PREFECTURAL PUBLIC UNIV CORP [JP]) 7 May 2014 (2014-05-07) * paragraph [0019] - paragraph [0039] * * figures 8B, 11, 20 * | 4,6,14, 15 | |
| Y | CROIZAT GAUTHIER ET AL: "A general framework for non-exponential delayed fluorescence and phosphorescence decay analysis, illustrated on Protoporphyrin IX", JOURNAL OF PHOTOCHEMISTRY AND PHOTOBIOLOGY B: BIOLOGY, ELSEVIER SCIENCE S.A., BASEL, CH, vol. 209, 30 April 2020 (2020-04-30), XP086218946, ISSN: 1011-1344, DOI: 10.1016/J.JPHOTOBIOL.2020.111887 [retrieved on 2020-04-30] * the whole document * | 5,7,8,15 | |

TECHNICAL FIELDS
SEARCHED     (IPC)

G01N

-/--

The present search report has been drawn up for all claims

| Place of search | Date of completion of the search | Examiner |
|---|---|---|
| Munich | 14 October 2020 | Sauerer, Christof |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

 

& : member of the same patent family, corresponding
    document

EPO FORM 1503 03.82 (P04C01)

page 1 of 2

Europäisches
Patentamt

European
Patent Office

Office européen
des brevets

# EUROPEAN SEARCH REPORT

Application Number

EP 20 17 6067

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MARK A WESTON ET AL: "Monitoring oxygen concentration during photodynamic therapy using prompt photosensitizer fluorescence", PHYSICS IN MEDICINE AND BIOLOGY, INSTITUTE OF PHYSICS PUBLISHING, BRISTOL GB, vol. 58, no. 20, 20 September 2013 (2013-09-20), pages 7039-7059, XP020251896, ISSN: 0031-9155, DOI: 10.1088/0031-9155/58/20/7039 [retrieved on 2013-09-20] * the whole document * ----- | 1-16 | |
| A,D | IVO S. VINKLÁREK ET AL: "Singlet oxygen feedback delayed fluorescence of protoporphyrin IX in organic solutions", PHOTOCHEMICAL AND PHOTOBIOLOGICAL SCIENCES, vol. 16, no. 4, 24 November 2016 (2016-11-24), pages 507-518, XP055738973, GB ISSN: 1474-905X, DOI: 10.1039/C6PP00298F * the whole document * ----- | 1-16 | TECHNICAL FIELDS SEARCHED (IPC) |

The present search report has been drawn up for all claims

| Place of search | Date of completion of the search | Examiner |
|---|---|---|
| Munich | 14 October 2020 | Sauerer, Christof |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 20 17 6067

This annex lists the patent family members relating to the patent documents cited in the above-mentioned European search report.
The members are as contained in the European Patent Office EDP file on
The European Patent Office is in no way liable for these particulars which are merely given for the purpose of information.

14-10-2020

| Patent document cited in search report | | Publication date | Patent family member(s) | | Publication date |
|---|---|---|---|---|---|
| EP 1742038 | A1 | 10-01-2007 | AT | 524725 T | 15-09-2011 |
| | | | EP | 1742038 A1 | 10-01-2007 |
| | | | EP | 1904831 A1 | 02-04-2008 |
| | | | EP | 2273255 A1 | 12-01-2011 |
| | | | US | 2009130700 A1 | 21-05-2009 |
| | | | US | 2010255523 A1 | 07-10-2010 |
| | | | WO | 2007004873 A1 | 11-01-2007 |
| EP 2728344 | A1 | 07-05-2014 | CN | 103608662 A | 26-02-2014 |
| | | | EP | 2728344 A1 | 07-05-2014 |
| | | | JP | 5991975 B2 | 14-09-2016 |
| | | | JP | WO2013002350 A1 | 23-02-2015 |
| | | | KR | 20140039030 A | 31-03-2014 |
| | | | US | 2014163391 A1 | 12-06-2014 |
| | | | WO | 2013002350 A1 | 03-01-2013 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1742038 A
- WO 200700487 A
- WO 2007004873 A
- WO 2010019041 A
- WO 201001041 A

**Non-patent literature cited in the description**

- **EPEL.** Oxygen-Guided Radiation Therapy. *Int J Radiat Oncol Biol Phys.,* 2019, vol. 103 (4), 977-984
- **WAGNER et al.** *Free Radical Biology and Medicine,* 2011, vol. 51 (3), 700-712
- **SIEGEMUND et al.** *Intens Care Med,* 1999, vol. 25, 1044-1060
- **J.M. VANDERKOOI et al.** *Am J Physiol,* 1991, vol. 260, C1131-50
- **VANDERKOOI et al.** *J Biol Chem,* 1987, vol. 262, 5476-5482
- **VANDERKOOI et al.** *Science,* 1987, vol. 236, 568-569
- **SINAASAPPEL ; INCE.** *J Appl Physiol,* 1996, 2297-2303
- **SINAASAPPEL.** *J Physiol,* 1999, vol. 514, 245-253
- **VANDERKOOI.** *Biochim Biophys Acta,* 1989, vol. 976, 1-27
- **BALZANI et al.** Photochemistry and Photophysics. Concepts, Research, Applications. *Angew. Chemie Int. Ed.,* 2014, vol. 53, 8817-8817
- **LAKOWICZ.** The principles of fluorescence spectroscopy. Springer, 2006, 954
- **MIK et al.** *Nature Methods,* 2006, vol. 3, 939-945
- **MIK et al.** *Biophys. J.,* 2008, vol. 95 (8), 3977-90
- **VINKLAREK et al.** *hotochem. Photobiol.,* 2017, vol. 16, 507-518
- **VINKLAREK et al.** *Photochem. Photobiol.,* 2017, vol. 16, 507-518
- **HARMS et al.** *Mitochondrion,* 2013, vol. 13, 507-514
- **PAPKOVSKY DB et al.** *J. of Fluo,* 2005, vol. 15 (4), 569-584
- **GEDDES CD et al.** Reviews in fluorescence. Springer, 2005, vol. 2, 125-151
- **ZITOVA A et al.** *Analytical Biochemistry,* 2010, vol. 397 (2), 144-151
- **DMITRIEV R ; PAPKOVSKY D.** *Cell. Mol. Life Sci.,* 2012, vol. 69, 2025-2039
- **SARDER et al.** *Bioconjug Chem.,* 2015, vol. 26 (6), 963-974
- **V. HUNTOSOVA et al.** *Journal of Photochemistry and Photobiology B: Biology,* 2016, vol. 164, 49-56
- **CHANTRELL et al.** *Journal of Luminescence,* 1976, vol. 12-13, 887-891
- **SCHOLZ ; DEDIC.** *Singlet-Oxygen-sensitized Delayed Fluorescence,* 2016, 63-81
- **PARKER ; HATCHARD.** *Transactions of the Faraday Society,* 1963, vol. 59, 284
- **PARKER.** Photoluminescence of Solutions: With Applications to Photochemistry and Analytical Chemistry. Elsevier Publishing Group University of California, 1968, 544
- **MIK et al.** *Anesthesia and Analegesia,* 2013, vol. 117, 834-846
- **GOUTERMAN et al.** *Journal of Molecular Spectroscopy,* 1974, vol. 53 (1), 88-100
- **S. JACQUES.** Optical Properties of Biological Tissues: A Review. *Physics in Medicine and Biology,* 2013, vol. 58 (11), R37-R61
- **TEWARI et al.** *Photochemical & Photobiological Sciences,* 2018, vol. 17, 1553-1572
- **WU et al.** *ACS Appl Mater Interfaces,* 2017, vol. 9 (17), 14596-14605
- **LI et al.** *European Jounral of Medicinal Chemistry,* 2019, vol. 181, 111582
- **SUISALU et al.** Phosphorescence-detected triplet state microwave resonance of a protoporphyrin molecule in n-octane. *Chem. Phys. Lett.,* 1983, vol. 101, 182-186
- **GOUTERMAN et al.** Porphyrin free base phosphorescence. *J. Mol. Spectrosc.,* 1974, vol. 53, 88-100
- **KUBAT et al.** *ACS Applied Materials and Interfaces,* 2017, vol. 9, 36229-36238
- **BIRKS et al.** *Spectrochimica Acta,* 1966, vol. 22, 323-331
- **SCHOLZ et al.** *Photochemical & Photobiological Sciences,* 2013, vol. 12, 1873

- **SCHOLZ et al.** *Photochemical & Photobiological Sciences,* 2017, vol. 16, 1643-1653

- **SCHOLZ et al.** *Photochemical & Photobiological Sciences,* 2015, vol. 14, 700-713